(19) Europäisches Patentamt / European Patent Office / Office européen des brevets

(11) **EP 4 071 238 A1**

(12) **EUROPEAN PATENT APPLICATION**
published in accordance with Art. 153(4) EPC

(43) Date of publication:
**12.10.2022 Bulletin 2022/41**

(21) Application number: **20851313.5**

(22) Date of filing: **20.08.2020**

(51) International Patent Classification (IPC):
**C12N 5/077** (2010.01)    **A01N 1/02** (2006.01)
**A61K 35/34** (2015.01)    **A61P 9/00** (2006.01)
**C12M 3/00** (2006.01)

(52) Cooperative Patent Classification (CPC):
**A61P 9/10; A61K 35/34; C12N 5/0657;**
C12N 2500/38; C12N 2501/415; C12N 2501/727;
C12N 2513/00; C12N 2533/90

(86) International application number:
**PCT/KR2020/011077**

(87) International publication number:
**WO 2021/112374 (10.06.2021 Gazette 2021/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.12.2019 KR 20190158572**
**23.07.2020 KR 20200091585**

(71) Applicant: **T&R Biofab Co., Ltd.**
**Siheung-si, Gyeonggi-do 15073 (KR)**

(72) Inventors:
• **MOON, Sung Hwan**
  **Guri-si, Gyeonggi-do 11940 (KR)**
• **PARK, Soon Jung**
  **Seoul 02638 (KR)**
• **JUNG, Taek Hee**
  **Yongin-si, Gyeonggi-do 16834 (KR)**
• **BAN, Ki Won**
  **Gimpo-si, Gyeonggi-do 10080 (KR)**

(74) Representative: **Plougmann Vingtoft a/s**
**Strandvejen 70**
**2900 Hellerup (DK)**

(54) **CARDIOMYOCYTE AGGREGATE PRODUCTION TECHNIQUE IMPROVING SURVIVAL RATE UNDER CRYOPRESERVATION AND HYPOXIC CONDITIONS**

(57) The present invention relates to a method for increasing the efficiency of long-term storage of cardiomyocyte clusters. Enabling the cryopreservation of cardiomyocyte clusters, the method of the present invention makes a contribution to the expansion of research into 3D cell structures such as the fabrication of organoids and the development of new drugs using organoids as well as increasing cell viability of cardiomyocytes in hypoxia after transplantation in vivo, with the expectation of finding advantageous applications in the related field including cell therapy products.

【Fig. 19】

1 aggregate ≒ 41±3 cells    1 aggregate ≒ 105±5 μm
200 μm

EP 4 071 238 A1

**Description**

Technical Field

The present invention was supported by the Korean Ministry of Health and Welfare under project No. HI20C0184010020. The research management organization for this project is the Korean Health Industry Development Institute, the title of the research enterprise is "Development of Advanced Medical Technology (R&D), the title of the research project is "Search for technology for maturation induction through microenvironment control, and convergence prototype development", the supervision institution is T&R Biofab, and the research period is April 23, 2020 to December 31, 2022.

The present invention was supported by the Korea Disease Control and Prevention Agency under project No. 2020-ER6106-00. The research management organization for this project is the Korea Disease Control and Prevention Agency, the title of the research enterprise is "Construction of Infrastructure for National Health and Medical Research (R&D)", the title of the research project is "Establishment of Drug Response Data for Cardiomyocytes originating from pluripotent stem cell lines of National Stem Cell Bank", the supervision institution is T&R Biofab, and the research period is March 16, 2020 to December 31, 2021.

The present invention was supported by the Ministry of Trade, Industry, and Energy under project No. 20009748. The research management organization for this project is the Korea Evaluation Institute of Industrial Technology, the title of the research enterprise is "Platform Construction of New Drug Development Based on 3D Biotissue Chip (Multi-Agency)-Commercialization of 3D Biotissue Chip", the title of the research project is "Development of 3D Cardiac Microenvironment-Simulating Chip Based on Human Stem Cell-Derived Cardiomyocyte for Assessing Cardiotoxicity of Drug", the supervision institution is the CHA University Industry Academy Cooperation Foundation, and the research period is April 01, 2020 to December 31, 2023.

This application claims priority to and the benefit of Korean Patent Application No. 10-2019-0158572, filed on December 02, 2019 and Korean Patent Application No. 10-2020-0091585, filed on July 23, 2020, the disclosures of which are incorporated herein by reference in their entireties.

The present invention relates to a technique for fabrication of cardiomyocyte clusters having improved viability under cryopreservation and hypoxic conditions. The cardiomyocyte clusters fabricated according to the present invention are highly viable in a hypoxic condition, can be cryopreserved for a long period of time, and show very excellent engraftment rates and heart function improvements when transplanted to a heart even after long-term cryopreservation.

Background Art

Patients suffering from incurable heart diseases, such as acute myocardial infarction, which are rapidly increasing worldwide in incidence, are treated by surgery or medication therapy. With conventional approaches, however, cell therapy has not been advanced for effectuating the regeneration of damaged myocardium.

Totipotent stem cells are cells that remain undifferentiated, with the capacity to develop into various cells constituting bio-tissues. Thus, many studies in the regenerative medicine field utilize stem cells, with the expectation of application to the treatment of diseases or tissue damage.

For cardiomyocytes to which totipotent stem cells are induced to differentiate, studies have been focused on the injection of cell clusters, rather than single cells, in order to effectuate maximal therapeutic effects. Such cardiomyocyte clusters are known to increase paracrine signaling, thus exhibiting large therapeutic effects when injected into the human body.

Conventional fabrication methods for clusters include a hanging-drop method, a rotary culture method using a bioreactor, a centrifugation method, a method using magnetic particles, a micromolding method, etc. When injected in vivo, clusters that are not uniform in size undergo internal cell necrosis due to hypoxia. Therefore, it is important to fabricate clusters that are uniform in size and are highly likely to survive hypoxia

In addition, conventional three-dimensional cell culturing methods do not show a way to cryopreserve cell clusters thus obtained, that is, a way to freeze and thaw the cell clusters without decreasing cell viability.

Therefore, the development of cell clusters that can be frozen and thawed allows the expansion of study ranges for 3D cell scaffolds and is expected to fabricate organoids with totipotent stem cells and to commercially supply clusters to new drug developers that utilize organoids. When injected in vivo, furthermore, the clusters are expected to provide information for researchers of cell therapy products because of their high viability in a hypoxic condition.

Detailed Description of the Invention

Technical Problem

Leading to the present invention, thorough and intensive research, conducted by the present inventors, into the development of a fabrication method for cardiomyocyte clusters that are possible to freeze and thaw, resulted in the finding that when formed in uniform size, cardiomyocyte clusters increase in viability without internal necrosis caused by hypoxia as well as maintaining viability even after being frozen and thawed.

Therefore, a purpose of the present invention is to provide a method for fabrication of a cryopreservable cardiomyocyte cluster.

Another purpose of the present invention is to provide a cryopreservable cardiomyocyte cluster.

A further purpose of the present invention is to provide a pharmaceutical composition comprising a cryopreservable cardiomyocyte cluster.

Technical Solution

The present inventors have conducted thorough and intensive research into the development of a fabrication method for cardiomyocyte clusters that are possible to freeze and thaw, finding that when formed in uniform size, cardiomyocyte clusters increase in viability without internal necrosis caused by hypoxia as well as maintaining viability even after being frozen and thawed.

Below, a detailed description will be given of the present invention.

An aspect of the present invention is drawn to a method for fabrication of a cluster of cardiomyocytes derived from human totipotent stem cells, the method comprising the steps of:

culturing human totipotent stem cells to differentiate into cardiomyocytes;
selecting CD71 positive cardiomyocytes, which express the CD71 surface marker, from the differentiated cardiomyocytes, to form a population of specific cardiomyocytes; and
forming a cluster ranging in diameter from 100 to 300 $\mu$m from the selected specific cardiomyocytes through cultivation.

As used herein, a "stem cell" refers to a cell that possesses self-renewal and the potential to differentiate into two or more different cell types.

In an embodiment of the present invention, the stem cell may be selected from a human embryonic stem cell and a reprogrammed stem cell.

In an embodiment of the present invention, the stem cell may be an autologous or homologous stem cell and may be derived from any animal including human and non-human mammals, and may be derived from an adult or an embryo. For example, the stem cell may be selected from the group consisting of an embryonic stem cell, an adult stem cell, an induced pluripotent stem cell (iPSC), and an iPSC-derived mesenchymal stromal cell, but is not limited thereto.

As used herein, the term "adult stem cell" refers to an undifferentiated cell, found in umbilical cord blood or adult bone marrow, blood, etc., which is in a state just before differentiation into specialized organ cells, with the potency to readily develop into a tissue in the body, as needed.

In an embodiment of the present invention, the adult stem cell may be selected from an adult stem cell of human, or animal tissue origin, a human-, or animal tissue-derived mesenchymal stromal cell, and a mesenchymal stromal cell derived from an induced pluripotent stem cell of human or animal tissue origin, but is not limited thereto.

In the present invention, the human or animal tissue may be selected from umbilical cord, umbilical cord blood, bone marrow, fat, muscle, nerve, skin, amnion, and placenta, but without limitations thereto.

In the present invention, the stem cells of various human or animal origins may be selected from the group consisting of hematopoietic cells, mammary stem cells, intestinal stem cells, endothelial progenitor cells, neural stem cells, olfactory stem cells, and spermatogonial stem cells, but are not limited thereto.

As used herein, the term "embryonic stem cell" refers to an in vitro culture of stem cells derived from the inner mass of a blastocyst, which is? an embryo before implantation of a fertilized egg into the uterus of the mother's body.

Embryonic stem cells are capable of self-renewal and possess pluripotency or totipotency to produce all types of cells in any tissue of an organism and, in a broad sense, are intended to encompass embryoid bodies.

In the present invention, the stem cells may include embryonic stem cells from any origin, such as humans, monkeys, pigs, horses, cows, sheep, dogs, cats, mice, rabbits, etc., but without limitations thereto.

As used herein, the term "induced pluripotent stem cell" (iPSC), also known as "reprogrammed cell", refers to a cell that has been induced from differentiated cells by artificial dedifferentiation to possess pluripotency.

The artificial dedifferentiation process can be performed by using a virus-mediated vector such as retrovirus and lentivirus or a non-viral vector or by introduction of non-virus-mediated dedifferentiation factors using proteins and cell extracts, and includes a dedifferentiation process by stem cell extracts, compounds, or the like.

Induced pluripotent stem cells have properties almost similar to those of embryonic stem cells, and specifically, show similarity in cell morphology and expression patterns of gene and protein, have pluripotency in vitro and in vivo, form teratomas, and generate chimeric mouse and germline transmission after blastocyst injection.

In the present invention, the CD71 marker may be used as a surface marker expressed specifically on contracting cardiomyocytes. When stained for the CD71 markers, cardiomyocytes that do not express CD71, that is, CD71-negative cardiomyocytes may be undifferentiated stem cells, embryoid bodies, or non-contracting cardiomyocytes.

According to the method of the present invention, when differentiation from stem cells into cardiomyocytes does not occur or when there are non-contracting cardiomyocytes in spite of the differentiation, the cardiomyocytes that do not exhibit contractility can be simply excluded with the CD71 marker. In addition, the CD71 marker may be used to form a homogenous aggregate that consists of contracting cardiomyocytes and does not include cardiomyocytes exhibiting different characteristics.

In an embodiment of the present invention, the specific cardiomyocyte population in the selecting step may include CD71-positve cardiomyocytes in an amount of 40% or more, 45% or more, 50% or more, 55% or more, 60% or more, 65% or more, 70% or more, 75% or more, 80% or more, 85% or more, 90% or more, 95% or more, 99% or more, 99.9% or more, or 99.99% or more, for example, 90% or more, but with no limitations thereto.

In the selecting step according to an embodiment of the present invention, CD71-positve cardiomyocytes may be separated by flow cytometry or manually.

In the forming step according to an embodiment of the present invention, the specific cardiomyocytes may be cultured to form a cluster with a diameter of 85 to 125 $\mu$m, 90 to 120 $\mu$m, 95 to 115 $\mu$m, or 100 to 110 $\mu$m, for example, 100 to 110 $\mu$m, but are not limited thereto.

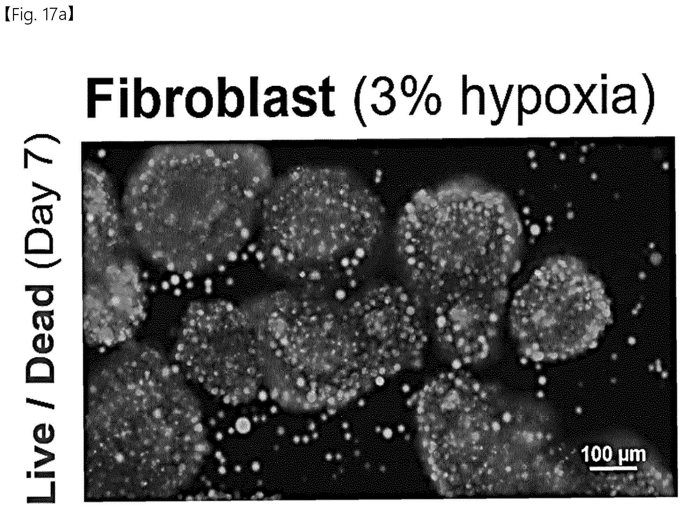

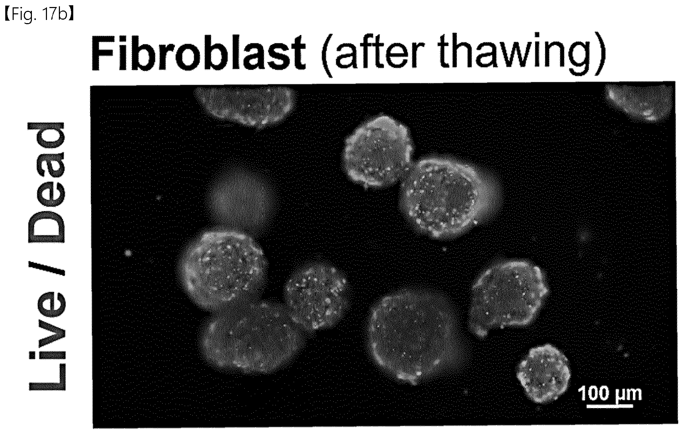

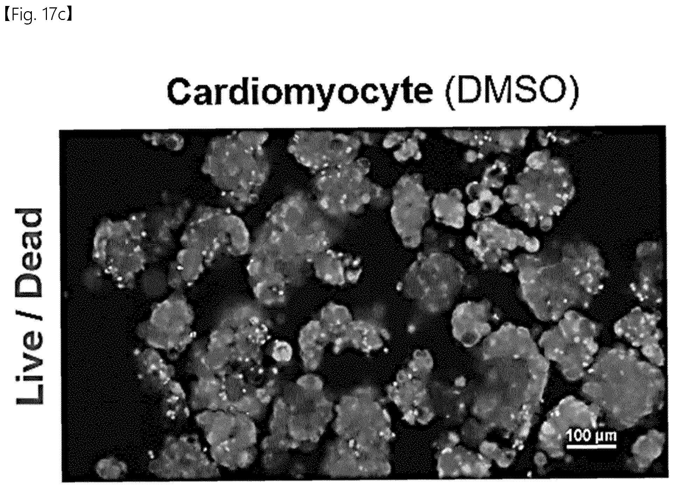

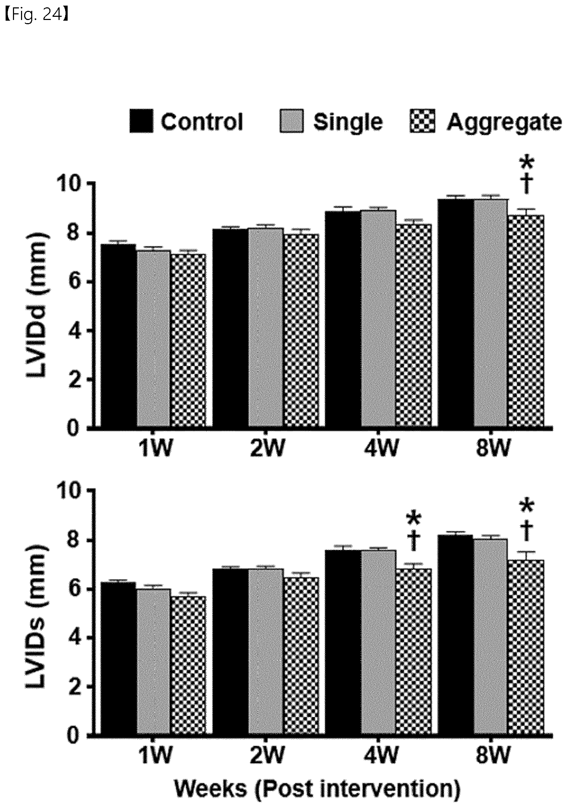

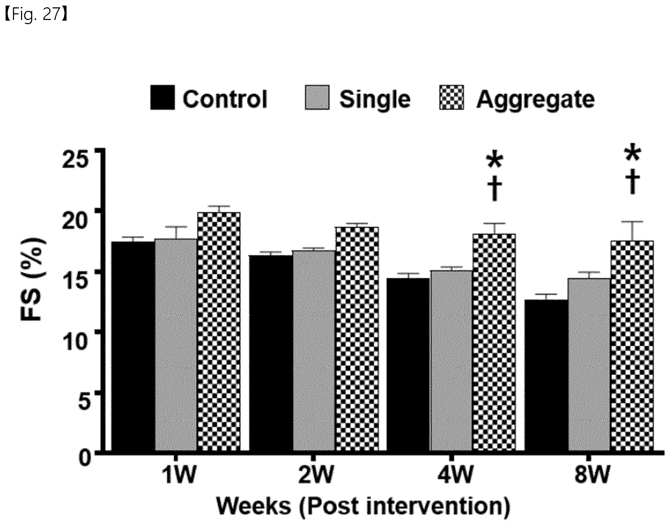

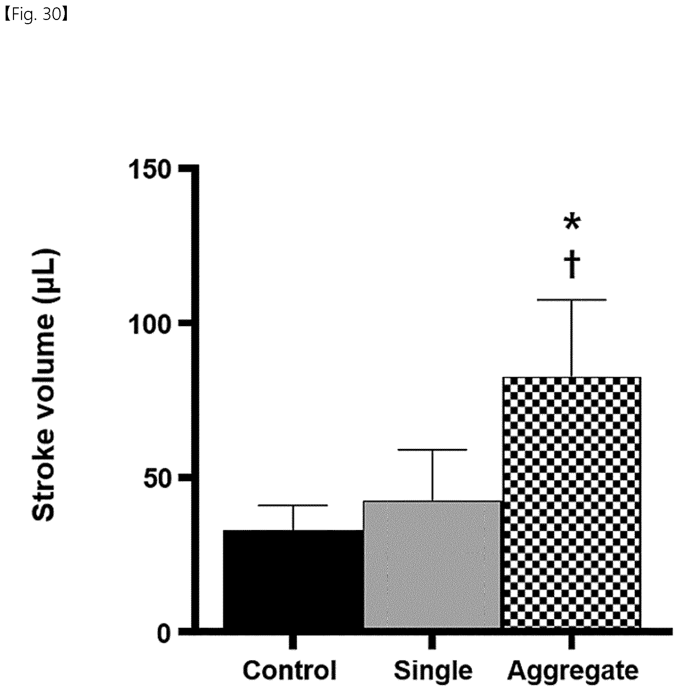

Given an excessive diameter, the cluster may be induced to undergo internal apoptosis upon hypoxia or long-term cryopreservation, with the resultant decrease of cell viability in the cluster. The cluster which has too small a diameter to differ from single cells becomes viable only at a low rate upon injection into the body, thus exhibiting a remarkably decreased effect of improving cardiac functions (see FIGS. 17a and 19 to 30) .

In an embodiment of the present invention, the forming step may be carried out by culturing the specific cardiomyocytes in a spheroid forming dish (SFD) to form a cluster.

In the present invention, the spheroid forming dish may have a diameter of 170 to 230 $\mu$m, from 175 to 225 $\mu$m, 180 to 220 $\mu$m, 185 to 215 $\mu$m, 190 to 210 $\mu$m, 195 to 205 $\mu$m, or 200 $\mu$m, for example, 200 $\mu$m, but with no limitations thereto.

In an embodiment of the present invention, the forming step may be carried out by culturing the specific cardiomyocytes in a spheroid forming dish having a diameter of 170 to 230 $\mu$m, 175 to 225 $\mu$m, 180 to 220 $\mu$m, 185 to 215 $\mu$m, 190 to 210 $\mu$m, 195 to 205 $\mu$m, or 200 $\mu$m to form a cardiomyocyte cluster ranging in diameter from 85 to 125 $\mu$m, from 90 to 120 $\mu$m, from 95 to 115 $\mu$m, or from 100 to 110 $\mu$m.

In an embodiment of the present invention, the forming step may be carried out by culturing the specific cardiomyocytes in a spheroid forming dish having a diameter of 170 to 230 $\mu$m to form a cardiomyocyte cluster ranging in diameter from 85 to 125 $\mu$m.

In an embodiment of the present invention, the forming step may be carried out by culturing the specific cardiomyocytes in a spheroid forming dish having a diameter of 170 to 230 $\mu$m to form a cardiomyocyte cluster ranging in diameter from 90 to 120 $\mu$m.

In an embodiment of the present invention, the forming step may be carried out by culturing the specific cardiomyocytes in a spheroid forming dish having a diameter of 170 to 230 $\mu$m to form a cardiomyocyte cluster ranging in diameter from 100 to 110 $\mu$m.

In an embodiment of the present invention, the forming step may be carried out by culturing the specific cardiomyocytes in a spheroid forming dish having a diameter of 180 to 220 $\mu$m to form a cardiomyocyte cluster ranging in diameter from 85 to 125 $\mu$m.

In an embodiment of the present invention, the forming step may be carried out by culturing the specific cardiomyocytes in a spheroid forming dish having a diameter of 180 to 220 $\mu$m to form a cardiomyocyte cluster ranging in diameter from 90 to 120 $\mu$m.

In an embodiment of the present invention, the forming step may be carried out by culturing the specific cardiomyocytes in a spheroid forming dish having a diameter of 180 to 220 $\mu$m to form a cardiomyocyte cluster ranging in diameter from 95 to 115 $\mu$m.

In an embodiment of the present invention, the forming step may be carried out by culturing the specific cardiomyocytes in a spheroid forming dish having a diameter of 180 to 220 $\mu$m to form a cardiomyocyte cluster ranging in diameter from 100 to 110 $\mu$m.

In an embodiment of the present invention, the forming step may be carried out by culturing the specific cardiomyocytes in a spheroid forming dish having a diameter of 190 to 210 $\mu$m to form a cardiomyocyte cluster ranging in diameter from 85 to 125 μm.

In an embodiment of the present invention, the forming step may be carried out by culturing the specific cardiomyocytes in a spheroid forming dish having a diameter of 190 to 210 μm to form a cardiomyocyte cluster ranging in diameter from 90 to 120 μm.

In an embodiment of the present invention, the forming step may be carried out by culturing the specific cardiomyocytes in a spheroid forming dish having a diameter of 190 to 210 μm to form a cardiomyocyte cluster ranging in diameter from 95 to 115 μm.

In an embodiment of the present invention, the forming step may be carried out by culturing the specific cardiomyocytes in a spheroid forming dish having a diameter of 190 to 210 μm to form a cardiomyocyte cluster ranging in diameter from 100 to 110 μm.

When used in forming cell clusters, the spheroid forming dish with a predetermined diameter may confer uniform sizes on the cardiomyocyte clusters formed. Therefore, the use of a spheroid forming dish may allow the fabrication of uniform cell clusters at high yield in a standardized pattern, whereby the cardiomyocytes can retain stable viability even in a hypoxic condition or even after cryopreservation and can survive with high engraftment rates after transplantation into the body.

In the forming step according to an embodiment of the present invention, a cardiomyocyte cluster may be formed using 30 to 50, 32 to 48, 34 to 46, 36 to 44, or 38 to 44 single cardiomyocytes, for example, 38 to 44 single cardiomyocytes, but with no limitations thereto.

In an embodiment of the present invention, the method may further comprise a step of freezing the formed cluster for cryopreservation.

In the freezing step according to an embodiment of the present invention, the cluster may be cryopreserved for 6 months or longer.

In the freezing step according to an embodiment of the present invention, the cluster may be cryopreserved in a 10% DMSO solution.

In an embodiment of the present invention, the method may further comprise a step of thawing the frozen cluster.

In an embodiment of the present invention, the thawing step may include suspending the cluster in a low-glucose DMEM medium.

Another aspect of the present invention contemplates a cardiomyocyte cluster including CD71 surface marker-expressing cardiomyocytes derived from a human totipotent stem cell and having a diameter of 100 to 300 μm.

In an embodiment of the present invention, the human totipotent stem cell may be selected from a human embryonic stem cell and a human induced pluripotent stem cell.

In an embodiment of the present invention, the cardiomyocyte cluster may range in diameter from 85 to 125 μm, from 90 to 120 μm, from 95 to 115 μm, or from 100 to 110 μm, for example, from 100 to 110 μm, but with no limitations thereto.

In an embodiment of the present invention, the cardiomyocyte cluster may include CD surface marker-expressing cardiomyocytes in an amount of 80%.

Another aspect of the present invention provides a pharmaceutical composition comprising CD71 surface marker-expressing cardiomyocytes derived from human totipotent stem cells as an active ingredient for alleviation, suppression, prevention, or treatment of a heart disease, wherein the cardiomyocytes in the form of a cardiomyocyte cluster have a diameter of 100 to 300 μm.

Since the pharmaceutical composition according to the present invention comprises the same human totipotent stem cell-derived cardiomyocytes as in the aforementioned cardiomyocyte cluster, the common content therebetween is omitted in order to avoid undue redundancy leading to the complexity of the description.

The term "comprising as an active ingredient", as used herein, refers to containing a sufficient amount of exosomes isolated from stem cells or a culture thereof to achieve activity to alleviate, suppress, prevent, or treat a specific disease.

In an embodiment of the present invention, the heart disease may be selected from the group consisting of angina pectoris, myocardial infarction, valvular disease, cardiac failure, cardiac hypertrophy, arrhythmia, pericarditis, and endocarditis, but is not limited thereto.

In an embodiment of the present invention, the heart disease may be ischemic heart disease.

As used herein, the term "ischemic heart disease" is given to an ischemia caused by the reduction of blood flow to the heart muscle due to narrowed coronary arteries that supply blood to the heart. Angina pectoris and myocardial infarction are representative of ischemic heart disease and may lead to cardiac arrest in a serious case. A patient with ischemic heart disease suffers from chest pain as a typical symptom. With the progression of ischemic heart disease, the heart undergoes cardiac dysfunction, which may be developed into heart failure with dyspnea and arrhythmia. Atherosclerosis has been cited the main cause of ischemic heart disease.

In an embodiment of the present disclosure, the ischemic heart disease may be selected from the group consisting of angina pectoris, myocardial infarction, and heart failure, but is not limited thereto.

In the present invention, the pharmaceutical composition may comprise a pharmaceutically acceptable carrier, for example, lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but without limitations thereto.

In the present invention, the pharmaceutical composition may further comprise a lubricant, a humectant, a sweetener, a flavorant, an emulsifier, a suspending agent, a preservative, etc.

In the present invention the pharmaceutical composition may be administered orally or parenterally, for example, intracardially, intravenously, subcutaneously, intramuscularly, intraperitoneally, topically, intranasally, intrapulmonarily, intrarectally, intradurally, intraocularly, intradermally, and percutaneously, but without limitations thereto.

A suitable dose of the pharmaceutical composition of the present invention may vary depending on pharmaceutical formulation methods, administration modalities, the patient's age, body weight, sex, severity of diseases, diet, administration time, administration route, an excretion rate, and responsiveness. A dose effective for desirable treatment or prevention may be determined or prescribed. For instance, the pharmaceutical composition of the present invention may be administered at a daily dose of 0.0001-100 mg/kg.

The pharmaceutical compositions of the present invention may be prepared in unit dosage form by formulating with a pharmaceutically acceptable carrier and / or excipient, according to methods which can be easily carried out by those skilled in the art. It may also be prepared by incorporation into a multi-dose container. The formulations here may be in the form of solutions, suspensions or emulsions in oils or aqueous media, or in the form of extracts, powders, granules, tablets or capsules, and may further comprise dispersants or stabilizers, with no limitations thereto.

The dosage amount of the pharmaceutical composition of the present invention may vary depending on the patient's age, body weight, and sex, dosage form, health condition and severity of disease, and it may be divisionally administered once to several times a day at regular time intervals according to the decision of the practitioner or pharmacist. For example, the daily dose may be 1 to 1,000 mg/kg, based on the content of active ingredient. The above described doses are exemplified as an average case, and its dose may increase or decrease depending on personal differences.

Another aspect of the present invention provides a method for treatment or alleviation of heart disease, the method comprising a step of:

administering to a subject a composition comprising CD71 surface marker-expressing cardiomyocytes derived from human totipotent stem cells as an active ingredient, wherein the cardiomyocytes in the form of a cardiomyocyte cluster have a diameter of 100 to 300 $\mu$m.

Since the treatment method according to the present invention contemplates the same cardiomyocyte cluster as in the aforementioned pharmaceutical composition, the common content therebetween is omitted in order to avoid undue redundancy leading to the complexity of the description.

As used herein, the term "treatment" is intended to refer to any action resulting in an improvement in the symptoms of a disease or the beneficial alteration of the symptoms by administration of the composition.

As used herein, the term "administration" refers to provision of a substance to a patient in an appropriate manner. So long as it reaches a target tissue, any general administration route may be employed, such as oral or parenteral routes. In addition, the composition of the present invention can be administered by any device that is able to deliver the active ingredient to the target cells.

The term "subject", as used herein, is intended to encompass, for example, humans, monkey, cow, horses, sheep, pigs, chickens, turkeys, quails, cats, dogs, mice, rats, rabbits, or guinea pigs, particularly humans, but with no limitation

In an embodiment of the present invention, the pharmaceutical composition may be administered alone or in combination of a pharmaceutically acceptable carrier that is selected in consideration of general administration routes and the standard pharmaceutical practice.

Another aspect of the present invention pertains to a use of a composition comprising CD71 surface marker-expressing cardiomyocytes derived from human totipotent stem cells as an active ingredient, wherein the cardiomyocytes in the form of a cardiomyocyte cluster have a diameter of 100 to 300 $\mu$m.

Advantageous Effect

The present invention relates to a method for increasing the efficiency of long-term storage of cardiomyocyte clusters. Enabling the cryopreservation of cardiomyocyte clusters, the method of the present invention makes a contribution to the expansion of research into 3D cell structures such as the fabrication of organoids and the development of new drugs using organoids as well as increasing cell viability of cardiomyocytes in hypoxia after transplantation in vivo, with the expectation of finding advantageous applications in the related field including cell therapy products.

Brief Description of the Drawings

FIG. 1 is a schematic diagram showing a differentiation procedure of human embryonic stem cells (hESCs) and human induced pluripotent stem cells (hiPSCs) according to an embodiment of the present invention.

FIG. 2 shows photographic images illustrating the expression of GFP gene on day 8 after hESC differentiation according to an embodiment of the present invention.

FIG. 3 shows photographic images illustrating a flow cytometry sorting procedure of GFP positive (GFP+) cardiomyocytes differentiated from hESC according to an embodiment of the present invention.

FIG. 4 shows photographic images illustrating a manual procedure for isolating contracting GFP+ cardiomyocytes after flow cytometry according to an embodiment of the present invention.

FIG. 5a show a graph illustrating analysis results of main components in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 5b is a heatmap illustrating analysis results (a to d) of main components in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 5c shows a sector in the heatmap illustrating analysis results (a to d) of main components in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 5d shows b sector in the heatmap illustrating analysis results (a to d) of main components in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 5e shows c sector in the heatmap illustrating analysis results (a to d) of main components in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 5f shows d sector in the heatmap illustrating analysis results (a to d) of main components in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 6a is a heatmap illustrating analysis results of main components for cardiac muscle cell-specific gene expression in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 6b is a heatmap illustrating analysis results of main components for ventricular muscle cell-specific gene expression in undifferentiated cells, flow cytometry sorted cells (GFP(+/-)) and manually isolated cells (GFP+ contracting cardiomyocytes and GFP+ non-contracting cardiomyocytes) according to an embodiment of the present invention.

FIG. 7 is an image of KEGG pathways analyzed for GFP+ contracting cardiomyocytes according to an embodiment of the present invention.

FIG. 8 shows flow cytometry profiles illustrating expression patterns of CD71 in GFP+ contracting cardiomyocytes according to an embodiment of the present invention.

FIG. 9 is a photographic image illustrating the expression of CD71 in hESC-derived cardiomyocytes according to an embodiment of the present invention.

FIG. 10 shows photographic images of CD71 surface marker-expressing cardiomyocytes after immunohistochemical staining according to an embodiment of the present invention.

FIG. 11 shows electrical signals of CD71 surface marker-expressing cardiomyocytes as measured by electrophysiological analysis according to an embodiment of the present invention.

FIG. 12a is a profile illustrating cTnT expression levels in CD71 surface marker-expressing cardiomyocytes after long-term cultivation according to an embodiment of the present invention.

FIG. 12b shows photographic images illustrating morphologies of CD71 surface marker-expressing cardiomyocytes after long-term cultivation according to an embodiment of the present invention.

FIG. 12c shows an electrical signal of CD71 surface marker-expressing cardiomyocytes as measured by electrophysiological analysis after long-term cultivation according to an embodiment of the present invention.

FIG. 13 shows photographic images illustrating the observation of contracting cardiomyocytes 10 days after the differentiation of clinical grade CMC-11 iPSC cell line 17 according to an embodiment of the present invention.

FIG. 14 shows photographic images illustrating immunohistochemical staining results of sorted cardiomyocytes after sorting clinical grade CMC-11 iPSC cell line 17 by CD71 surface marker according to an embodiment of the present invention.

FIG. 15 is a graph illustrating viability of the single cardiomyocytes and the cardiomyocyte cluster after incubation in a hypoxic condition according to an embodiment of the present invention.

FIG. 16 shows photographic images of the single cardiomyocytes and the cardiomyocyte cluster after incubation in a hypoxic condition according to an embodiment of the present invention.

FIG. 17a is a photographic image illustrating the survival of fibroblast clusters against hypoxia according to an embodiment of the present invention.

FIG. 17b is a photographic image illustrating the survival of fibroblast clusters 300 $\mu$m or less in size upon thawing after cryopreservation according to an embodiment of the present invention.

FIG. 17c is a photographic image illustrating the survival of the cardiomyocytes upon thawing after cryopreservation in the presence of dimethyl sulfoxide (DMSO) according to an embodiment of the present invention.

FIG. 18 is a graph illustrating viability of cardiomyocyte clusters according to size upon thawing after cryopreservation according to an embodiment of the present invention.

FIG. 19 shows photographic images illustrating a procedure of forming cardiomyocyte clusters according to an embodiment of the present invention.

FIG. 20 shows a photographic image of a cardiomyocyte cluster observed to contract after cryopreservation, together with the electrophysiological function thereof according to an embodiment of the present invention.

FIG. 21a is a photographic image of 100 $\mu$m cardiomyocyte clusters that have passed through a 27G syringe according to an embodiment of the present invention.

FIG. 21b is a photographic image of 200 $\mu$m cardiomyocyte clusters that have passed through a 27G syringe according to an embodiment of the present invention.

FIG. 21c is a photographic image illustrating comparison of volumes according to cell counts in single cardiomyocytes and cardiomyocyte clusters according to an embodiment of the present invention.

FIG. 21d is a photographic image showing a procedure of injecting single cardiomyocytes and cardiomyocyte clusters into different sites after myocardial infarction (MI) according to an embodiment of the present invention.

FIG. 22a is a graph showing RWT measured in a control, a single cell-administered group, and a cluster-administered group according to an embodiment of the present invention.

FIG. 22b is a graph showing SWT measured in a control, a single cell-administered group, and a cluster-administered group according to an embodiment of the present invention.

FIG. 22c is a graph showing PWT measured in a control, a single cell-administered group, and a cluster-administered group according to an embodiment of the present invention.

FIG. 23 shows echocardiograms of myocardial infarction rats injected with PBS (control), $5\times10^6$ single cardiomyocytes (single cell-administered group) or a 105 $\pm$ 5 $\mu$m cluster of $2\times10^4$ cardiomyocytes (cluster-administered group) according to an embodiment of the present invention.

FIG. 24 shows graphs illustrating LVIDd and LVIDs measured in the control, the single cell-administered group, and the cluster-administered group according to an embodiment of the present invention.

FIG. 25 is a KaPLAN-Meier curve of the control, the single cell-administered group, and the cluster-administered group according to an embodiment of the present invention.

FIG. 26 is a graph showing LVEF measured in the control, the single cell-administered group, and the cluster-administered group according to an embodiment of the present invention.

FIG. 27 is a graph showing fractional shortening (FS) measured in the control, the single cell-administered group, and the cluster-administered group according to an embodiment of the present invention.

FIG. 28a shows pressure-volume loops of the heart in the control, the single cell-administered group, and the cluster-administered group as measured by hemodynamic assessment according to an embodiment of the present invention.

FIG. 28b shows pressure-volume loops of the heart in the control as measured by hemodynamic assessment according to an embodiment of the present invention.

FIG. 28c shows pressure-volume loops of the heart in the single cell-administered group as measured by hemodynamic assessment according to an embodiment of the present invention.

FIG. 28d shows pressure-volume loops of the cluster-administered group as measured by hemodynamic assessment according to an embodiment of the present invention.

FIG. 29 is a graph showing cardiac outputs (CO) measured in the control, the single cell-administered group, and the cluster-administered group according to an embodiment of the present invention.

FIG. 30 is a graph showing stroke volumes measured in the control, the single cell-administered group, and the cluster-administered group according to an embodiment of the present invention.

FIG. 31 is a graph showing maximum volumes (Vmax) measured in the control, the single cell-administered group, and the cluster-administered group according to an embodiment of the present invention.

FIG. 32a is a graph showing maximum pressures (Pmax) measured in the control, the single cell-administered group, and the cluster-administered group according to an embodiment of the present invention.

FIG. 32b is a graph showing dP/dt max measured in the control, the single cell-administered group, and the cluster-administered group according to an embodiment of the present invention.

FIG. 32c is a graph showing dP/dt min measured in the control, the single cell-administered group, and the cluster-administered group according to an embodiment of the present invention.

FIG. 33 is a graph showing ESPVR measured in the control, the single cell-administered group, and the cluster-administered group according to an embodiment of the present invention.

FIG. 34 is a graph showing EDPVR measured in the control, the single cell-administered group, and the cluster-administered group according to an embodiment of the present invention.

FIG. 35 is a graph showing ESPVR and EDPVR measured in the control according to an embodiment of the present invention.

FIG. 36 is a graph showing ESPVR and EDPVR measured in the single cell-administered group according to an embodiment of the present invention.

FIG. 37 is a graph showing ESPVR and EDPVR measured in the cluster-administered group according to an embodiment of the present invention.

FIG. 38 shows photographic images of the single-administered group after immunohistochemistry according to an embodiment of the present invention.

FIG. 39 shows photographic images of the cluster-administered group after immunohistochemistry according to an embodiment of the present invention.

FIG. 40 is a graph showing densities of cTnT expressing-Dil labeled cells (Dil+/cTnT+) in a left ventricular tissues of the single cell-administered group and the aggregate-administered group according to an embodiment of the present invention.

FIG. 41 is a graph showing TUNEL assay results of the single-administered group and the cluster-administered group according to an embodiment of the present invention.

FIG. 42 shows photographic images illustrating results of immunohistochemistry analysis with Dil, cTnT, GJA1, and DAPI on the cluster-administered group according to an embodiment of the present invention.

FIG. 43 shows photographic images illustrating results of immunohistochemistry analysis with Dil, ACTN2, GJA1, and DAPI on the cluster-administered group according to an embodiment of the present invention.

FIG. 44 shows photographic images illustrating results of immunohistochemistry analysis with Dil, cTnT, hMYH7, and DAPI on the cluster-administered group according to an embodiment of the present invention.

FIG. 45 shows photographic images illustrating results of immunohistochemistry analysis with Dil, ACTN2, hMYH7, and DAPI on the cluster-administered group according to an embodiment of the present invention.

FIG. 46 shows photographic images illustrating results of immunohistochemistry analysis with CD31, cTnT, and DAPI on the control, the single cell-administered group, and the cluster-administered group according to an embodiment of the present invention.

FIG. 47 is a graph showing capillary densities in an infarcted zone and a border zone of the heart in the control, the single cell-administered group, and the cluster-administered group according to an embodiment of the present invention.

Best Mode for Carrying Out the Invention

Provided is a method for fabrication of a cluster of cardiomyocytes derived from human totipotent stem cells, the method comprising the steps of:

culturing human totipotent stem cells to differentiate into cardiomyocytes;
selecting CD71 positive cardiomyocytes, which express the CD71 surface marker, from the differentiated cardiomyocytes, to form a population of specific cardiomyocytes; and
forming a cluster ranging in diameter from 100 to 300 $\mu$m from the selected specific cardiomyocytes through cultivation.

Mode for Carrying Out the Invention

Hereafter, the present invention will be described in detail by examples. The following examples are intended merely to illustrate the invention and are not construed to restrict the invention.

**EXAMPLE 1: Isolation of Human Totipotent Stem Cell-Derived Cardiomyocytes**

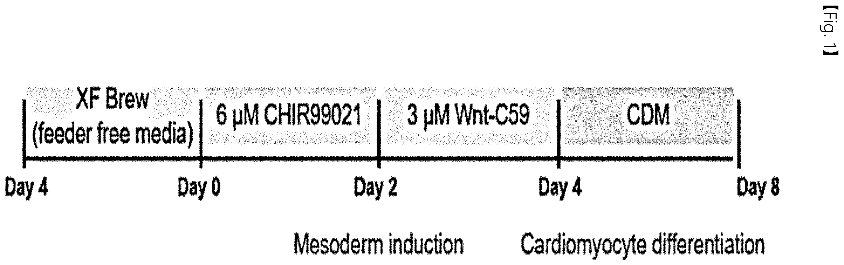

The human embryonic stem cell (hESC) line H9 (NKX2.5 eGFP/w) was expanded on Matrigel in iPS-BREW XF medium (StemMACS TM, Mitenyi Biotec) for four days without differentiation. For initial differentiation, the cultured stem cells were sequentially treated with CHIR 99021 and Wnt-C59 for 2 days for each, followed by incubation in cardiomyocyte differentiation medium (CDM; RPMI1640 (ThermoFisher Scientific) + 500 $\mu$g/ml Human serum albumin Sigma Aldrich) + 213 $\mu$g/ml Ascorbic acid (Sigma Aldrich)) with for 4 days to obtain contracting cardiomyocytes (FIG. 1).

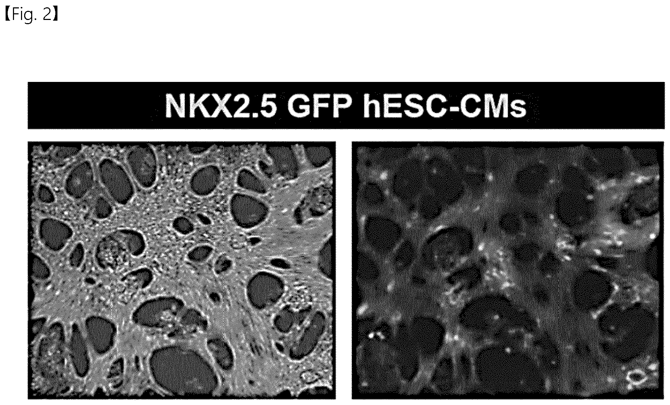

After cultivation for a total of 8 days, GFP+ contracting cardiomyocytes appeared, with the hESC line H9 observed to express the cardiomyocyte-specific gene NKX2.5 (NKX2.5 GFP+) (FIG. 2).

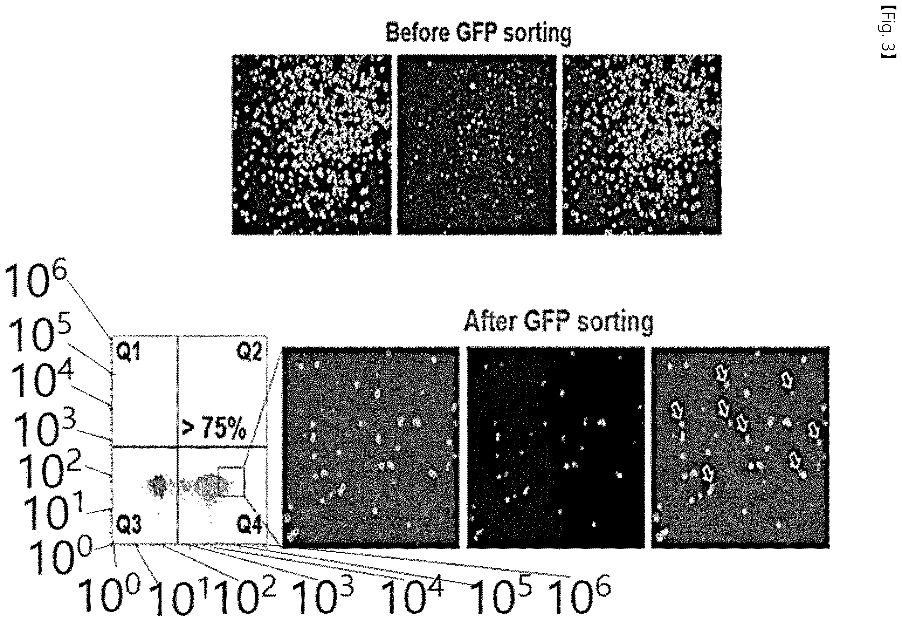

Subsequently, the differentiated cells were dissociated into single cells using Accutase (Gibco) and then washed with PBS. The dissociated cells were suspended in PBS containing 5 % fetal bovine serum (FBS, HyClone), and GFP+ cells were sorted through SH800S Cell Sorter flow cytometer with Cell sorter software Ver 2.1.2 (Sony Biotechnology) (FIG. 3).

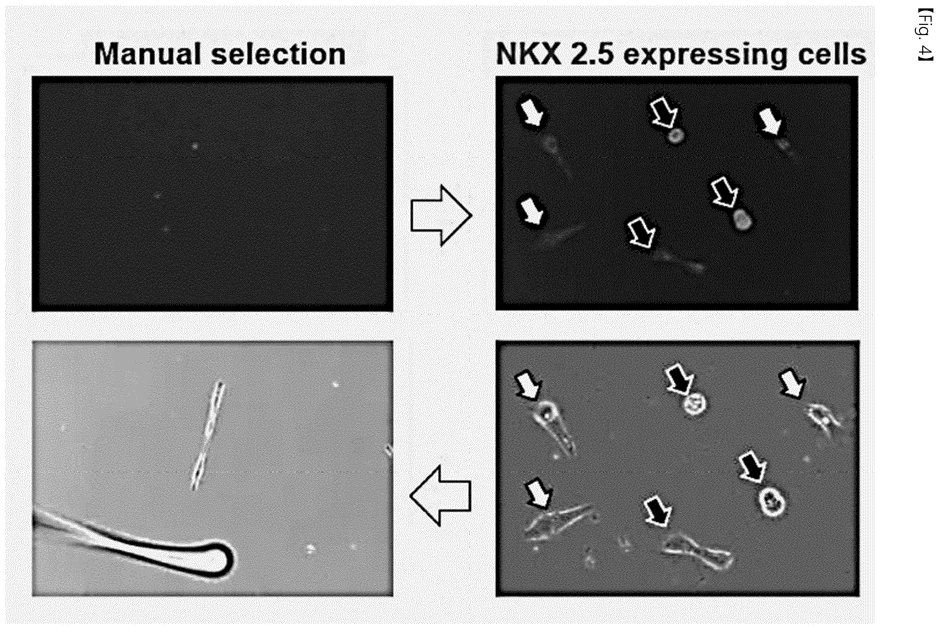

More than 75% or the cells were found to express GFP, as assayed by flow cytometry. After the flow cytometry sorting, GFP+ cells were isolated by manual pipetting from the heterogeneous culture under microscopic observation to establish a homogenous GFP+ cell population. In the sample, contracting cardiomyocytes and non-contracting cardiomyocytes coexisted. Subsequently, the GFP+ contracting cardiomyocytes were manually separated from the non-contracting cardiomyocytes again under microscopic observation (FIG. 4).

**EXAMPLE 2: Identification of Specific Surface Marker in Contracting Cardiomyocyte**

**2-1. Gene expression pattern analysis through RNA sequencing**

Transcription profiles were compared by single cell total RNA sequencing analysis between the manually separated contracting cardiomyocytes and the flow cytometry sorted cardiomyocytes.

For RNA sequencing, a final cDNA library was constructed from RNA isolated from each sample, using Illumina dye sequencing SMART-Seq® v4 Ultra®Low Input RNA kit according to the manufacturer's protocol. Then, the library was quantified by qPCR according to qPCR quantification protocol guide (KAPA Library Quantification kits for Illumina Sequencing platforms). The quality of the libraries was assessed using Agilent Technologies 4200 TapeStation (Agilent Technologies, Germany). The indexed libraries were sequenced on Illumina Hiseq2500 platform (Illumina, Inc., USA).

After sequencing, the data (reads) were aligned to Homo sapiens (hg19) using Tophat v2.0.13. and only the mapped read pairs were used for differentially expressed gene (DEG) analysis. For DEGs, fragments per kilobase of exon per million fragment (FPKM) values was used to analyze gene expression by CUFFLINKS software v.2.2.1. The data thus obtained was subjected to principal component analysis (PCA).

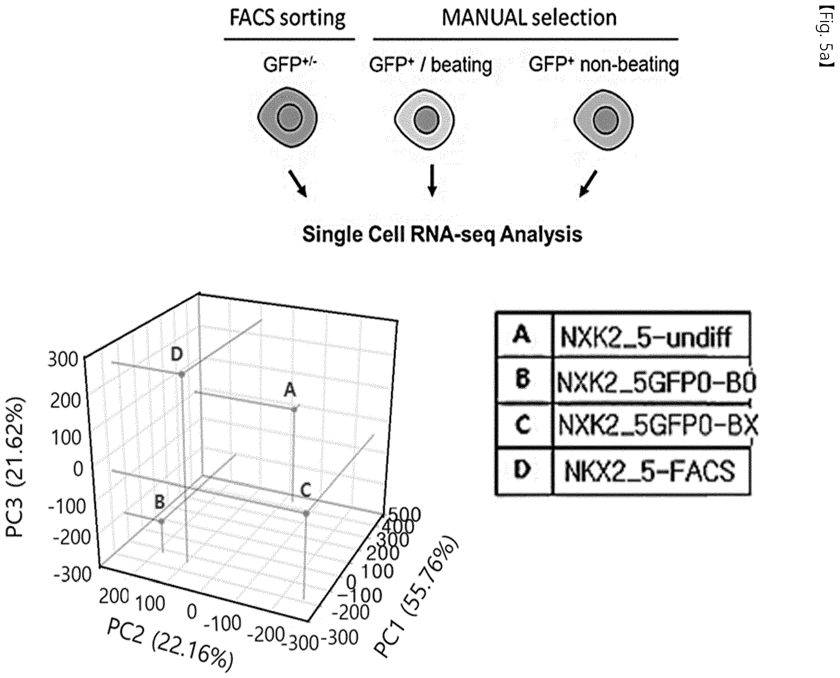

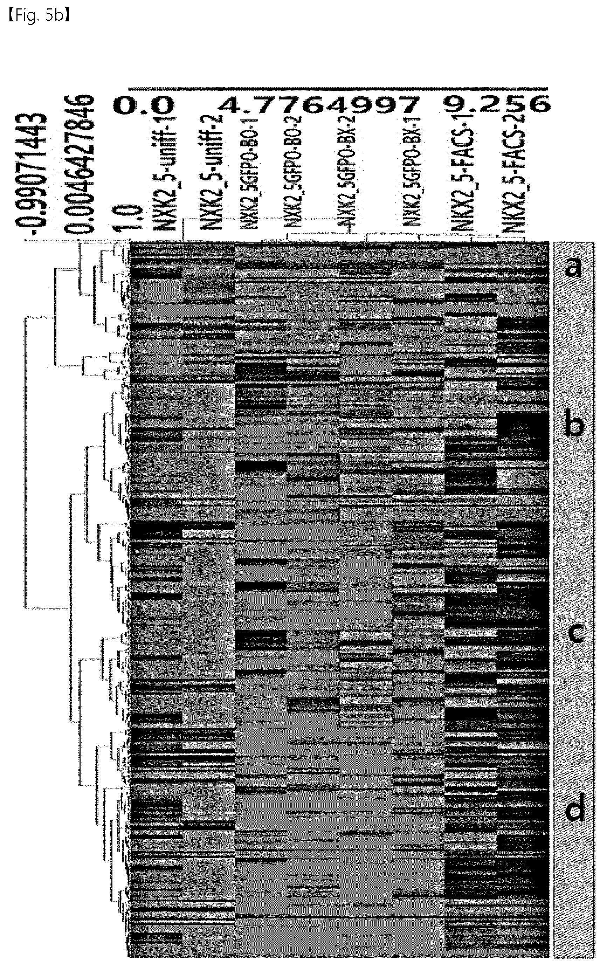

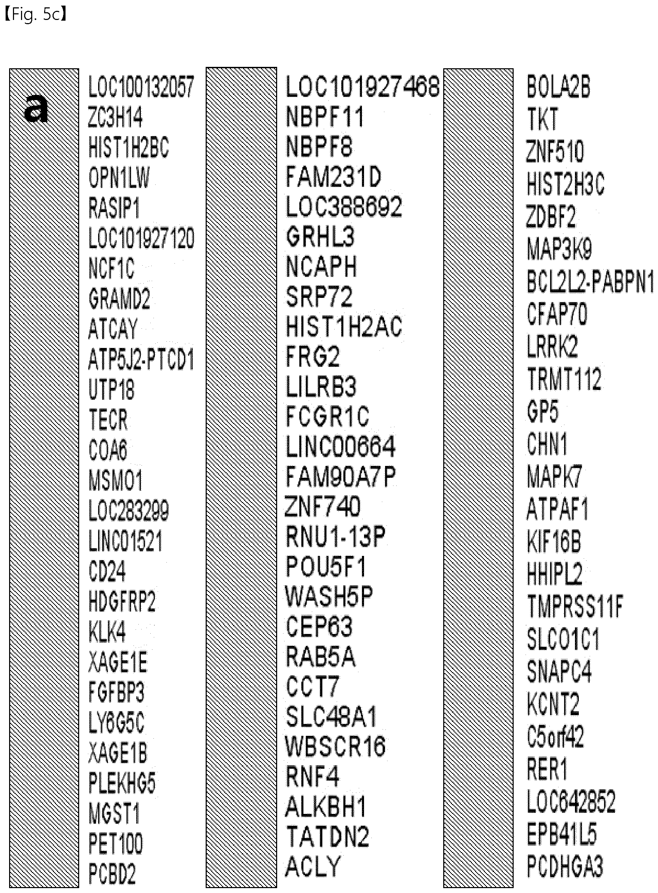

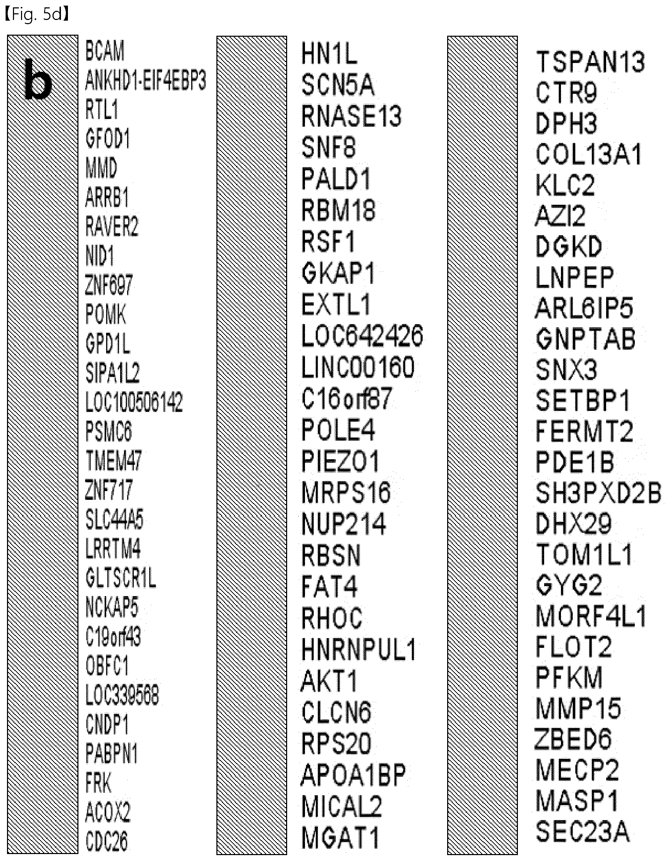

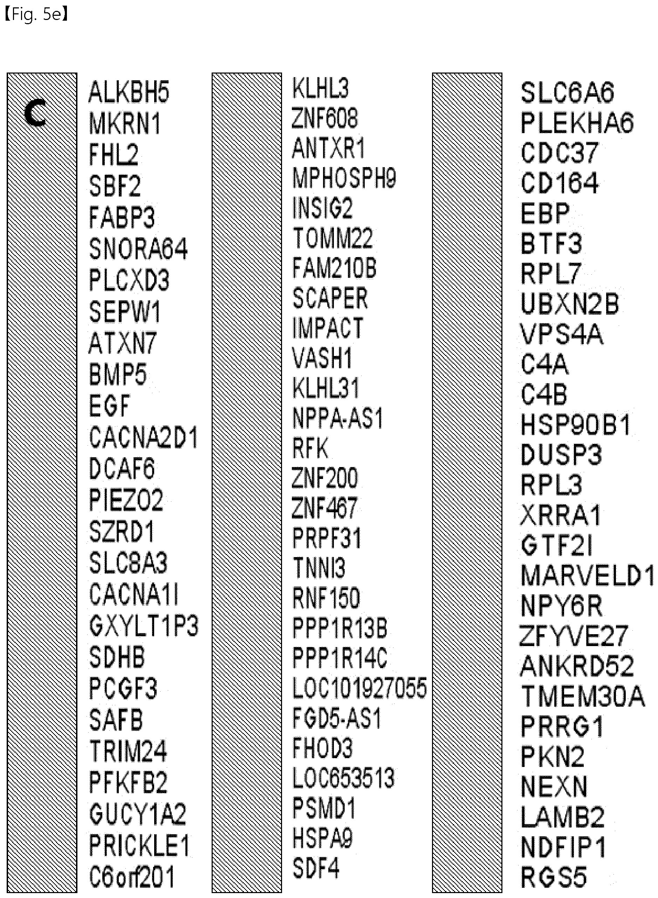

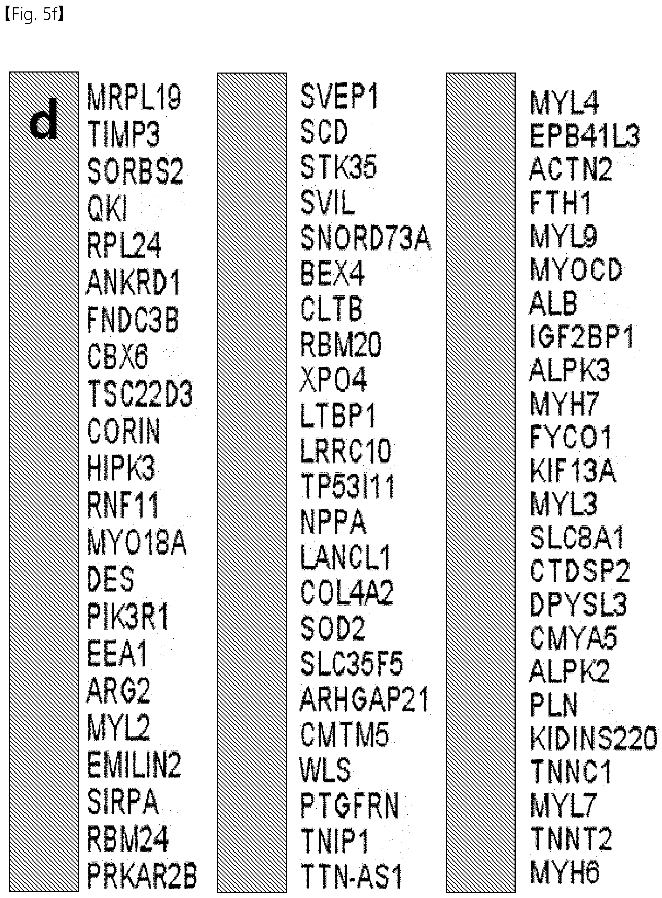

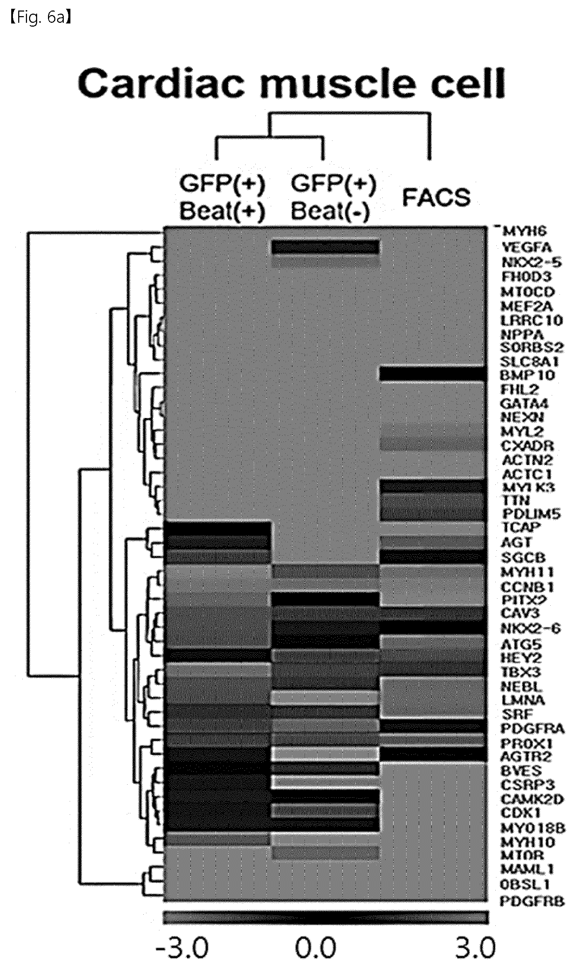

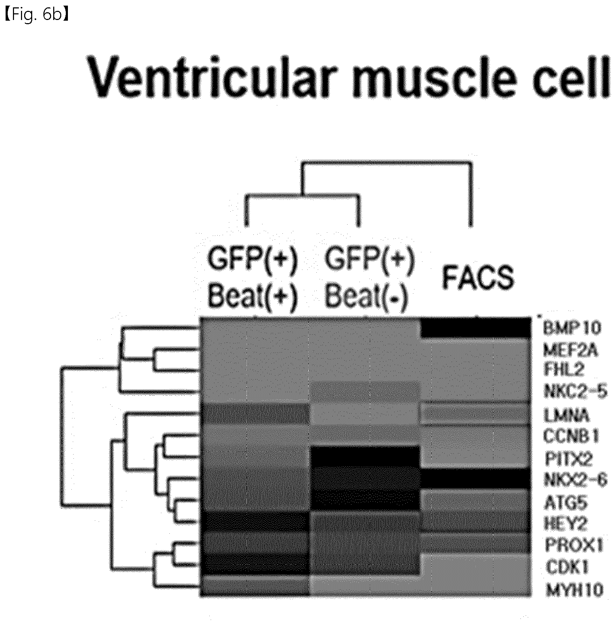

As a result of the analysis, the flow cytometry sorted cells (GFP(+/-)) and the manually separated cells (GFP+ contracting cardiomyocytes and GFP+ non-contracting cardiomyocytes) both were similar in characteristics to cardiomyocytes, with explicit discrimination from the undifferentiated cells. In addition, NKX 2.5 GFP+ contracting cells, exclusive of undifferentiated cells, were observed to exhibit certainly different expression patterns from the other cardiomyocyte groups (FIGS. 5a to 5f). In detail, the GFP+ contracting cells were high in the expression levels of genes specific for atrial myocytes and ventricular myocytes, thus demonstrating definite characteristics of cardiomyocytes (FIGS. 6a to 6b).

**2-2. Contracting cardiomyocyte-specific surface marker screening**

Based on the result of Example 2-1, GFP+ contracting cardiomyocytes were screened for surface markers.

Certain genes (GO: 0009986) were analyzed and 288 upregulated cell surface genes were isolated. Then, using STRING (http://string-db.org/), KEGG pathway enrichment analysis was performed (Table 1).

TABLE 1

| Term ID | Term description | Observed gene count | False discovery rate | Genes |
|---|---|---|---|---|
| hsa04 640 | Hemato poieti c cell lineag e | 30 | 2.18E-24 | ANPEP, CD14, CD1B, CD2, CD33, CD38, CD4, CD44, CD5, CD59, CD8A, EPO, EPOR, FCER2, FLT3LG, IL1R1, IL2RA, IL6R, IL7R, ITGA1, ITGA2, ITGA2B, ITGA3, ITGA4, ITGA6, ITGAM, ITGB3, MS4A1, TFRC, TNF |

(continued)

| Term ID | Term descri ption | Observed gene count | False discovery rate | Genes |
|---|---|---|---|---|
| hsa04 060 | Cytoki ne- cytoki ne recept or intera ction | 40 | 1.39E-22 | ACKR3,ACVR2A,BMPR1A,BMPR2,CC R1,CCR10,CCR2,CCR7,CCR9,CD27 , CX3CL1,CXCL9,CXCR3,CXCR5,EP O,EPOR,FAS, FASLG,FLT3LG,IFNG ,IL13,IL17A,IL17RB,IL17RC,IL 1R1,IL2RA,IL6R,IL7R,PDGFB,PD GFC, TGFB1,TGFBR2,TNF,TNFRSF1 0A,TNFRSF12A, TNFRSF14,TNFRSF 18,TNFRSF1A,TNFSF18,TNFSF4 |
| hsa04 514 | Cell adhesi on molecu les (CAMs) | 25 | 1.99E-15 | CD2,CD226,CD274,CD276,CD4,CD58,CD86,CD8A, CDH5,CTLA4,ITGA 4,ITGA6,ITGAM,ITGB1,NLGN1,NR CAM,PDCD1,PTPRC,SDC,SDC3,SEL L,SELP,SPN,TIGIT, VTCN1 |
| hsa05 205 | Proteo glycan s ir cancer | 25 | 1.68E-12 | CAV1,CAV3,CD44,CD63,FAS,FASL G, FZD1,FZD10,FZD4, FZD9,ITGA2,ITGB1,ITGB3,ITGB5 , PPP1CC,RHOA,SDC2,TGFB1,THBS 1,TLR2,TLR4,TNF, WNT3A,WNT5A, WNT6 |
| hsa05 418 | Fluid shear stress and athero sclero sis | 17 | 1.97E-08 | ACVR2A,BMPR1A,BMPR2,CAV1,CAV 3,CDH5,IFNG, IL1R1,ITGA2, ITGB3,PDGFB,PLAT,RHOA,SDC2,T NF, TNFRSF1A,TRPV4 |
| hsa04 151 | PI3K-Akt signal ing pathwa y | 26 | 2.50E-08 | EPO,EPOR,FASLG,FGF10,FLT3LG, IL2RA,IL6R,IL7R, ITGA1,ITGA2, ITGA2B,ITGA3,ITGA4,ITGA6,ITG B1,ITGB3,ITGB5,LPAR1,PDGFB,P DGFC, PKN1,PKN2,TGFA,THBS1,TL R2,TLR4 |
| hsa05 200 | Pathwa ys ir cancer | 31 | 6.74E-08 | EPO,EPOR,FAS,FASLG,FGF10,FLT 3LG, FZD1,FZD10,FZD4,FZD9,HHI P,IFNG,IL13,IL2RA,IL6R, IL7R, ITGA2,ITGA2B,ITGA3,ITGA6,ITG B1,LPAR1,PDGFB,RALA,RHOA,TGF A, TGFB1,TGFBR2,WNT3A,WNT5A,W NT6 |
| hsa04 810 | Regula tion of actin cytosk eleton | 18 | 8.76E-07 | CD14,FGF10,ITGA1,ITGA2,ITGA2 B, ITGA3,ITGA4,ITGA6,ITGAM,IT GB1,ITGB3,ITGB5,MYH9,PDGFB,P DGFC,PPP1CC, RHOA,SSH1 |
| hsa04 512 | ECM- recept or intera ction | 12 | 9.54E-07 | CD44,HMMR,ITGA1,ITGA2,ITGA2B , ITGA3,ITGA4,ITGA6,ITGB1,ITG B3,ITGB5,THBS1 |
| hsa05 165 | Human papill omavir us infect ion | 22 | 1.09E-06 | FAS, FASLG, FZD1, FZD10, FZD4, FZ D9,ITGA1,ITGA2,ITGA2B,ITGA3, ITGA4,ITGA6,ITGB1,ITGB3,ITGB 5,PSEN1,THBS1,TNF, TNFRSF1A,W NT3A,WNT5A,WNT6 |
| hsa04 145 | Phagos ome | 15 | 1.27E-06 | CD14,FCGR3A,ITGA2,ITGAM,ITGB 1,ITGB3,ITGB5,LAMP1,PLA2R1,S CARB1,TFRC, THBS1,TLR2,TLR4,V AMP3 |
| hsa04 510 | Focal adhesi on | 17 | 1.78E-06 | CAV1,CAV3,ITGA1,ITGA2,ITGA2B , ITGA3,ITGA4,ITGA6,ITGB1,ITG B3,ITGB5,PDGFB, PDGFC,PPP1CC, RHOA,THBS1,TLN1 |

(continued)

| Term ID | Term description | Observed gene count | False discovery rate | Genes |
|---------|------------------|---------------------|----------------------|-------|
| hsa05 410 | Hypert rophic cardio myopat hy (HCM) | 11 | 5.39E-06 | ITGA1,ITGA2,ITGA2B,ITGA3,ITG A4,ITGA6,ITGB1,ITGB3,ITGB5,T GFB1,TNF |
| hsa05 414 | Dilate d cardio myopat hy (DCM) | 11 | 1.06E-05 | ITGA1,ITGA2,ITGA2B,ITGA3,ITG A4,ITGA6,ITGB1,ITGB3,ITGB5,T GFB1,TNF |
| hsa04 610 | Comple ment and coagul ation cascad es | 10 | 2.59E-05 | C5AR1,CD46,CD59,F10,FGB,FGG, ITGAM,PLAT,PLG, SERPINA5 |
| hsa05 152 | Tuberc ulosis | 14 | 3.45E-05 | CD14,FCGR3A,IFNG,ITGAM,LAMP1 ,LBP, NOD2,PLA2R1,RHOA,TGFB1, TLR2,TLR4,TNF, TNFRSF1A |
| hsa05 144 | Malari a | 8 | 3.81E-05 | IFNG,SDC2,SELP,TGFB1,THBS1,T LR2,TLR4,TNF |
| hsa04 015 | Rap1 signal ing pathwa y | 15 | 4.11E-05 | FGF10,GRIN1,GRIN2B,ITGA2B,IT GAM, ITGB1,ITGB3,LPAR1,P2RY1, PDGFB,PDGFC,RALA, RHOA,THBS1,TLN1 |
| hsa04 612 | Antige n proces sing and presen tation | 9 | 4.26E-05 | CD4,CD8A,CTSB,HSPA2,HSPA5,IF NG, KLRD1,PDIA3,TNF |
| hsa05 140 | Leishm aniasi s | 9 | 6.27E-05 | FCGR3A,IFNG,ITGA4,ITGAM,ITGB 1,TGFB1,TLR2,TLR4,TNF |
| hsa05 412 | Arrhyt hmogen ic right ventri cular cardio myopat hy (ARVC) | 9 | 7.35E-05 | ITGA1,ITGA2,ITGA2B,ITGA3,ITG A4,ITGA6,ITGB1,ITGB3,ITGB5 |
| hsa05 166 | HTLV-I infect ion | 16 | 8.91E-05 | FZD1,FZD10,FZD4,FZD9,IL1R1,I L2RA,NRP1,PDGFB, TGFB1,TGFBR2 ,TLN1,TNF,TNFRSF1A,WNT3A,WNT 5A, WNT6 |
| hsa04 310 | Wnt signal ing pathwa y | 12 | 9.37E-05 | FZD1,FZD10,FZD4,FZD9,MMP7,PS EN1,RHOA, SFRP1,SFRP4,WNT3A,W NT5A,WNT6 |
| hsa04 611 | Platel et activa tion | 11 | 0.00012 | FGB,FGG,ITGA2,ITGA2B,ITGB1,I TGB3,P2RY1,P2RY12,PPP1CC,RHO A,TLN1 |
| hsa04 390 | Hippo signal ing pathwa y | 12 | 0.00015 | BMPR1A,BMPR2,FZD1,FZD10, FZD4 ,FZD9,PPP1CC, TGFB1,TGFBR2,WN T3A,WNT5A,WNT6 |

(continued)

| Term ID | Term descri ption | Observed gene count | False discovery rate | Genes |
|---|---|---|---|---|
| hsa04 350 | TGF-beta signal ing pathwa y | 9 | 0.00016 | ACVR2A,BMPR1A,BMPR2,IFNG,RHO A, TGFB1,TGFBR2,THBS1,TNF |
| hsa05 217 | Basal cell carcin oma | 8 | 0.00016 | FZD1,FZD10,FZD4,FZD9,HHIP,WN T3A,WNT5A,WNT6 |
| hsa05 321 | Inflam matory bowel diseas e (IBD) | 8 | 0.00016 | IFNG,IL13,IL17A,NOD2,TGFB1,T LR2,TLR4,TNF |
| hsa05 162 | Measles | 11 | 0.0002 | CD46,FAS,FASLG,HSPA2,IFNG,IL13,IL2RA, SLAMF1,TLR2,TLR4,TN FRSF10A |
| hsa04 360 | Axon guidan ce | 12 | 0.00042 | BMPR2,EPHB6,ITGB1,NRP1,PLXNB 2,RHOA, ROBO2,SEMA7A,SSH1,TRP C4,UNC5D,WNT5A |
| hsa05 226 | Gastri c cancer | 11 | 0.00042 | ABCB1,FGF10,FZD1,FZD10,FZD4, FZD9,TGFB1,TGFBR2,WNT3A,WNT5 A,WNT6 |
| hsa05 332 | Graft- versus -host diseas e | 6 | 0.00042 | CD86,FAS,FASLG,IFNG,KLRD1,TN F |
| hsa04 150 | mTOR signal ing pathwa y | 11 | 0.00043 | FZD1,FZD10,FZD4,FZD9,RHOA,SL C3A2,TNF, TNFRSF1A,WNT3A,WNT5 A,WNT6 |
| hsa05 142 | Chagas diseas e (Ameri can trypan osomia sis) | 9 | 0.00053 | FAS,FASLG,IFNG,TGFB1,TGFBR2, TLR2,TLR4,TNF, TNFRSF1A |
| hsa05 323 | Rheuma toid arthri tis | 8 | 0.00084 | CD86,CTLA4,IFNG,IL17A,TGFB1, TLR2,TLR4,TNF |
| hsa05 145 | Toxopl asmosi s | 9 | 0.00086 | HSPA2,IFNG,ITGA6,ITGB1,TGFB1 ,TLR2,TLR4,TNF, TNFRSF1A |
| hsa04 550 | Signal ing pathwa ys regula ting plurip otency of stem cells | 10 | 0.00099 | ACVR2A,BMPR1A,BMPR2,FZD1,FZD 10,FZD4,FZD9,WNT3A,WNT5A,WNT6 |
| hsa04 979 | Choles terol metabo lism | 6 | 0.0014 | ABCA1,ANGPTL3,LIPC,LPL,SCARB 1,SORT1 |
| hsa05 146 | Amoebi asis | 8 | 0.0015 | CD14,IFNG,IL1R1,ITGAM,TGFB1, TLR2,TLR4,TNF |

(continued)

| Term ID | Term description | Observed gene count | False discovery rate | Genes |
|---|---|---|---|---|
| hsa04 080 | Neuroa ctive ligand - recept or intera ction | 14 | 0.0016 | ADCYAP1R1,C5AR1,CHRNA4,GHRHR ,GHSR, GLRA1,GRIN1,GRIN2B,KIS S1R,LPAR1,MAS1, NTSR1,P2RY1,PLG |
| hsa04 380 | Osteoc last differ entiat ion | 9 | 0.0018 | FCGR3A,IFNG,IL1R1,ITGB3,LILR B2,TGFB1,TGFBR2,TNF,TNFRSF1A |
| hsa04 650 | Natura l killer cell mediat ed cytotoxicity | 9 | 0.0018 | FAS,FASLG,FCGR3A,IFNG,KLRD1, MICA,TNF, TNFRSF10A,ULBP2 |
| hsa04 660 | T cell recept or signal ing pathwa y | 8 | 0.0018 | CD4,CD8A,CTLA4,IFNG,PDCD1,PT PRC,RHOA,TNF |
| hsa04 934 | Cushin g's syndro me | 10 | 0.0018 | FZD1,FZD10,FZD4,FZD9,KCNK2,M EN1,SCARB1,WNT3A,WNT5A,WNT6 |
| hsa05 150 | Staphy lococc us aureus infect ion | 6 | 0.0018 | C5AR1,FCGR3A,FGG,ITGAM,PLG,S ELP |
| hsa04 659 | Th17 cell differ entiat ion | 8 | 0.0021 | CD4,IFNG,IL17A,IL1R1,IL2RA,I L6R,TGFB1,TGFBR2 |
| hsa05 134 | Legion ellosi s | 6 | 0.0021 | CD14,HSPA2,ITGAM,TLR2,TLR4,T NF |
| hsa05 330 | Allogr aft reject ion | 5 | 0.0021 | CD86,FAS,FASLG,IFNG,TNF |
| hsa04 010 | MAPK signal ing pathwa y | 14 | 0.0026 | CD14,FAS,FASLG,FGF10,FLT3LG, HSPA2,IL1R1,PDGFB,PDGFC,TGFA , TGFB1,TGFBR2,TNF,TNFRSF1A |
| hsa05 225 | Hepato cellul ar carcin oma | 10 | 0.0026 | FZD1,FZD10,FZD4,FZD9,TGFA,TG FB1,TGFBR2,WNT3A,WNT5A,WNT6 |
| hsa05 340 | Primar y immuno defici ency | 5 | 0.0026 | ADA,CD4,CD8A,IL7R,PTPRC |
| hsa04 621 | NOD-like recept or signal ing pathwa y | 10 | 0.0028 | ANTXR1,ANTXR2,CTSB,GPRC6A,NO D2,PSTPIP1,RHOA,TLR4,TNF,TRP V2 |
| hsa05 010 | Alzhei mer's diseas e | 10 | 0.003 | ADAM17,BACE2,FAS,GRIN1,GRIN2 B,IDE,LPL, PSEN1,TNF,TNFRSF1A |
| hsa05 132 | Salmon ella infect ion | 7 | 0.003 | CD14,IFNG,LBP,MYH9,PKN1,PKN2 ,TLR4 |

(continued)

| Term ID | Term description | Observed gene count | False discovery rate | Genes |
|---|---|---|---|---|
| hsa05 164 | Influe nza A | 10 | 0.003 | FAS,FASLG,FURIN,HSPA2,IFNG,P LG,TLR4,TNF, TNFRSF10A,TNFRSF 1A |
| hsa04 940 | Type I diabet es mellit us | 5 | 0.0032 | CD86,FAS,FASLG,IFNG,TNF |
| hsa04977 | Vitamin digest ion and absorp tion | 4 | 0.004 | CUBN,FOLH1,SCARB1,SLC46A1 |
| hsa04 672 | Intest inal immune networ k for IgA produc tion | 5 | 0.0045 | CCR10,CCR9,CD86,ITGA4,TGFB1 |
| hsa04 062 | Chemokine signal ing pathwa y | 10 | 0.0046 | CCR1,CCR10,CCR2,CCR7,CCR9,CX 3CL1,CXCL9,CXCR3,CXCR5,RHOA |
| hsa04 916 | Melano genesi s | 7 | 0.0062 | FZD1,FZD10,FZD4,FZD9,WNT3A,W NT5A,WNT6 |
| hsa05 100 | Bacter ial invasi on of epithe lial cells | 6 | 0.0063 | CAV1,CAV3,ITGB1,RHOA,SEPT12, SEPT2 |
| hsa05 320 | Autoim mune thyroi d disease | 5 | 0.0065 | CD86,CTLA4,FAS,FASLG,TPO |
| hsa05 133 | Pertus sis | 6 | 0.0069 | CD14,ITGAM,ITGB1,RHOA,TLR4,T NF |
| hsa04 620 | Toll-like recept or signal ing pathwa y | 7 | 0.0072 | CD14,CD86,CXCL9,LBP,TLR2,TLR 4,TNF |

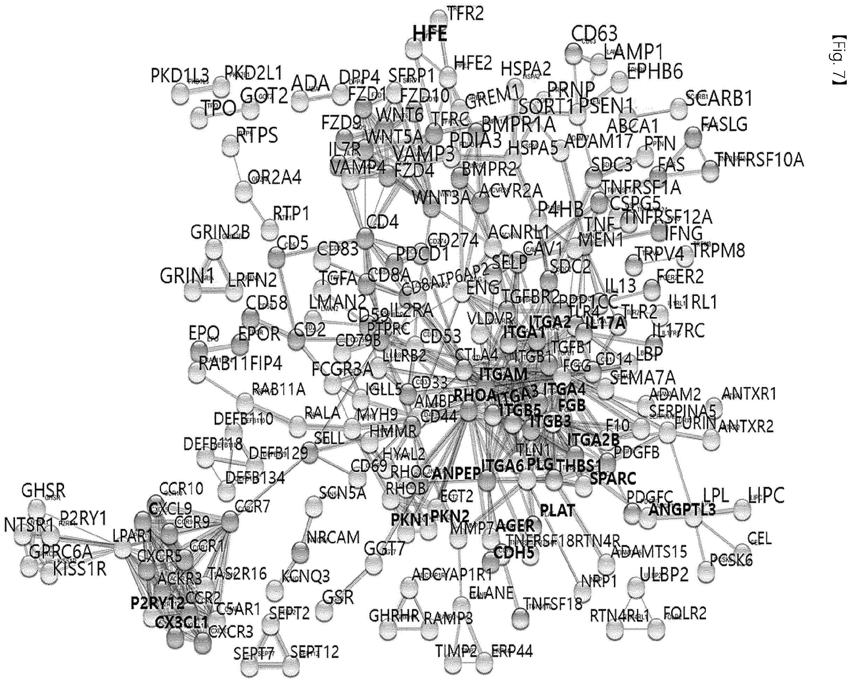

As a result, five KEGG pathways were found to have an FDR (false discovery rate) of 0.01 or less, with reliability of 0.17 or higher. The five KEGG pathways included a hematoblast lineage, cytokine-cytokine receptor interaction, cell adhesion molecules (CAMs), proteoglycans in cancer, and fluid shear stress and atherosclerosis. The KEGG pathways showed that the network of the 288 cell surface genes consists of 286 nodes and 666 edges (FIG. 7).

Among the 288 identified surface marker-related genes, the transferrin receptor (CD71) gene that is involved in left ventricular remodeling and induces myocardia in mouse KO model 28 was selected as a surface marker specific for contracting cardiomyocytes.

**EXAMPLE 3: Characterization of CD71 as Cardiomyocyte-Specific Surface Marker**

In order to examine whether CD71 can be used as a surface marker for filtering cardiomyocytes, hESC was analyzed for CD71 expression pattern during differentiation into cardiomyocytes.

**3-1. CD71 expression in contracting cardiomyocytes**

For CD71-expressing cardiomyocytes, flow cytometry and cell sorting were conducted on cardiomyocytes.

hESC-derived cardiomyocytes were suspended in PBS containing 1 % FBS and incubated with PE-Cy7-conjugated mouse anti-human SIRPα (Biolegend) and PE-conjugated mouse anti-human CD71 (Bioscience). Subsequently, cells were analyzed using an SH800S Cell Sorter flow cytometer (Sony Biotechnology) with Cell sorter software Ver 2.1.5 (Sony Biotechnology). For intracellular flow cytometry, cells were harvested with TrypLE Select (Gibco), fixed in 4% paraformaldehyde for 15 min at room temperature, blocked and permeablized in block buffer consisting of 1 × Perm/Wash Buffer (BD) and 4% goat serum for 15 min at 4 °C. Cells were then incubated with cTnT antibody (Abcam) for 1 hr at 4 °C and then Alexa Fluor 488-conjugated secondary (ThermoFisher Scientific, A-21235, diluted 1:1000) for 1 hr at 4 °C. For collection of flow cytometric data, cell sorting was done using an SH800S Cell Sorter flow cytometer (Sony Biotechnology) with Cell sorter software Ver 2.1.5 (Sony Biotechnology).

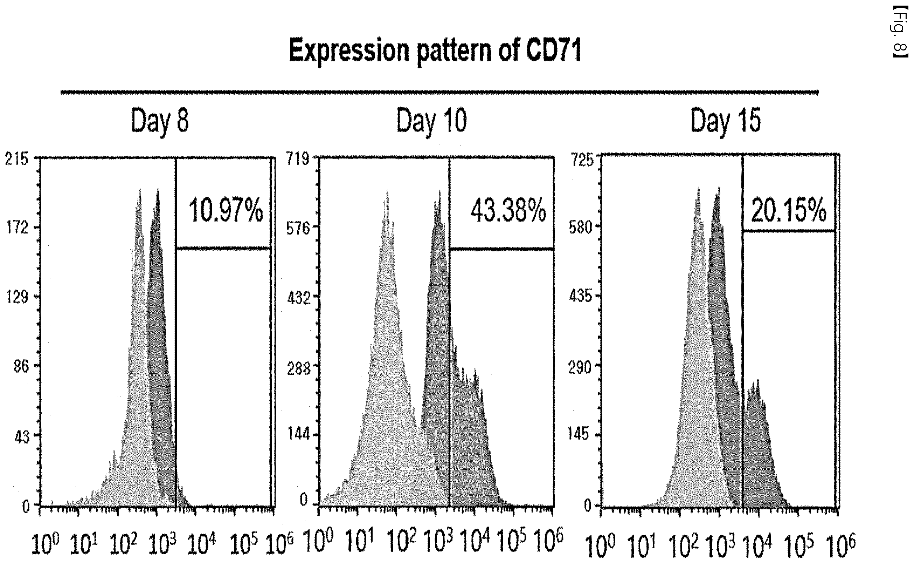

From the data, it was observed that CD71 expression peaked 10 days after differentiation at which contracting cardiomyocytes appeared, with the percentage thereof gradually decreasing with time (FIG. 8).

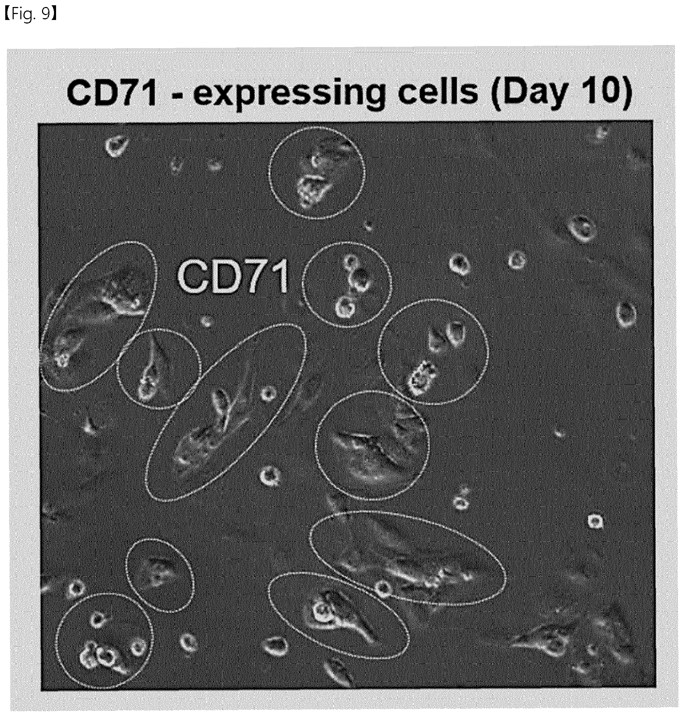

CD71 was found to be expressed specifically on the hESC-derived contracting cardiomyocytes (FIG. 9).

### 3-2. Characterization of CD71-expressing cardiomyocyte

After being separated by flow cytometry 10 days after differentiation as described in Example 3-1, the CD71-expressiong cells were subjected to immunochemical staining.

In brief, cells were fixed in 4% paraformaldehyde for 20 min and permeablized with 0.1 % Triton X-100 in phosphate buffered saline (PBS, Sigma) for 5 min. After blocking with 5 % normal goat serum for 30 min, the cells were stained with cTnT and α-actinin (Sigma-Aldrich) at 4°C for 12 hrs. The cells were washed three times with PBS and incubated with Alexa Fluor 488- or 555-conjugated secondary antibodies (ThermoFisher Scientific) for 1 hr while the nuclei were counterstained with DAPI (ThermoFisher Scientific). Imaging was performed on the fluorescence microscope Nikon TE2000-U (Nikon, Japan).

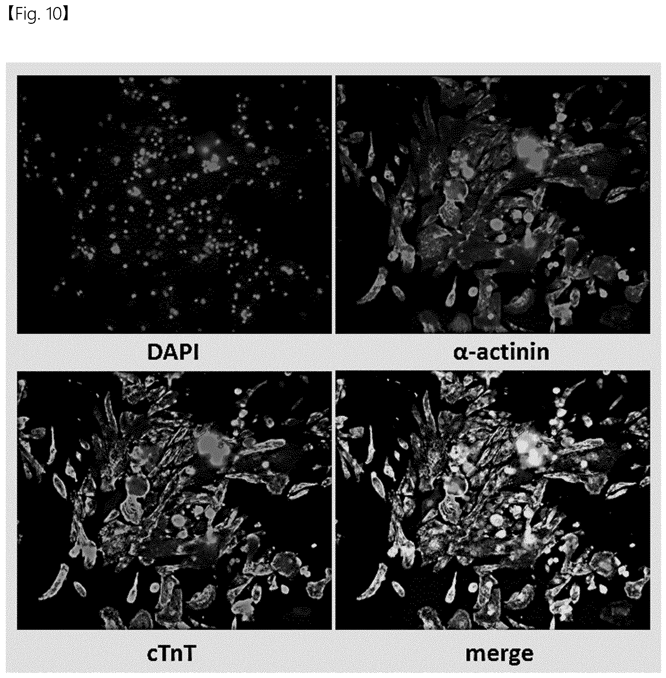

From the immunostaining results, it was discovered that the cells sorted by CD71 expressed cTnT and α-actin, which are markers specific for cardiomyocytes (FIG. 10).

### 3-3. Electrophysiological characterization of CD71-expressing cardiomyocytes

The cardiomyocytes sorted by CD71 marker in Example 3-1 were perfused with normal Tyrode solution containing 145mM NaCl, 4.3mM KCl, 1mM MgCl2, 1.8mM CaCl2, 10mM HEPES, and 5mM glucose at physiological temperature (37°C). In order to make a whole cell patch clamp condition, a glass capillary tube adapted to come into close contact with cell membrane was filled with internal pipette solution consisting of 120 mM K-aspartate, 20 mM KCl, 10 mM HEPES, 0.1 mM EGTA, 1 mM MgCl$_2$, 3 mM Mg-ATP, pH 7.25. Voltages and currents of the cardiomyocytes were recorded using Axopatch 200B amplifier with Digidata 1440A and pClamp software 10.1 (Axon Instruments, USA).

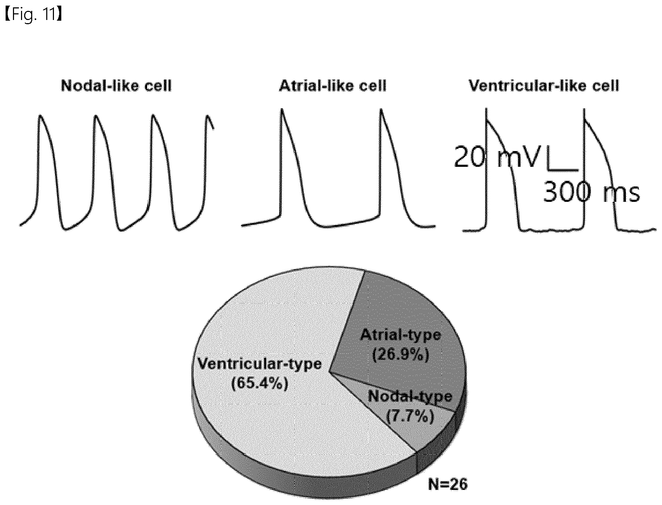

From the electrophysiological analysis data, it was observed that the CD71-expressing cells showed electrical signals of all of the three-type cardiomyocytes, with the most predominance of ventricular-like cells (≥65 %) (FIG. 11).

### 3-4. Characterization of CD71-expressing cardiomyocyte after in-vitro cultivation for long time

After being maintained in vitro for 70 days, the cardiomyocytes sorted by CD71 in Example 3-1 were characterized in the same manner as in Examples 3-1 to 3-3.

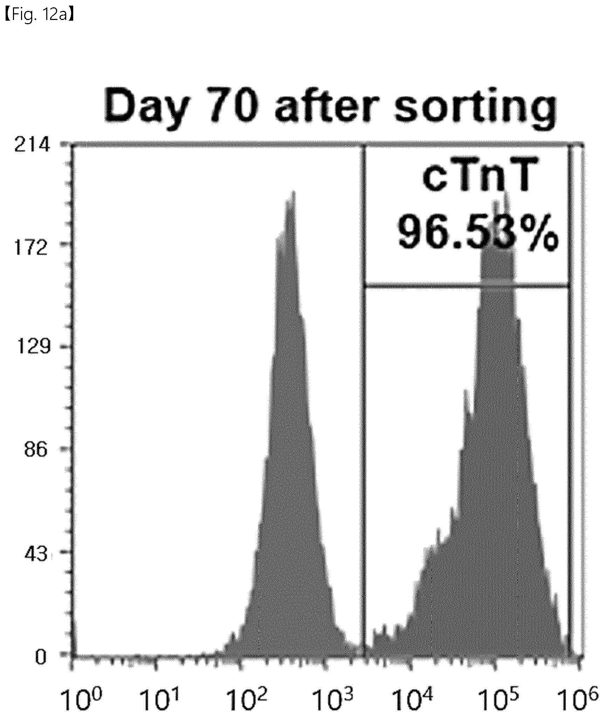

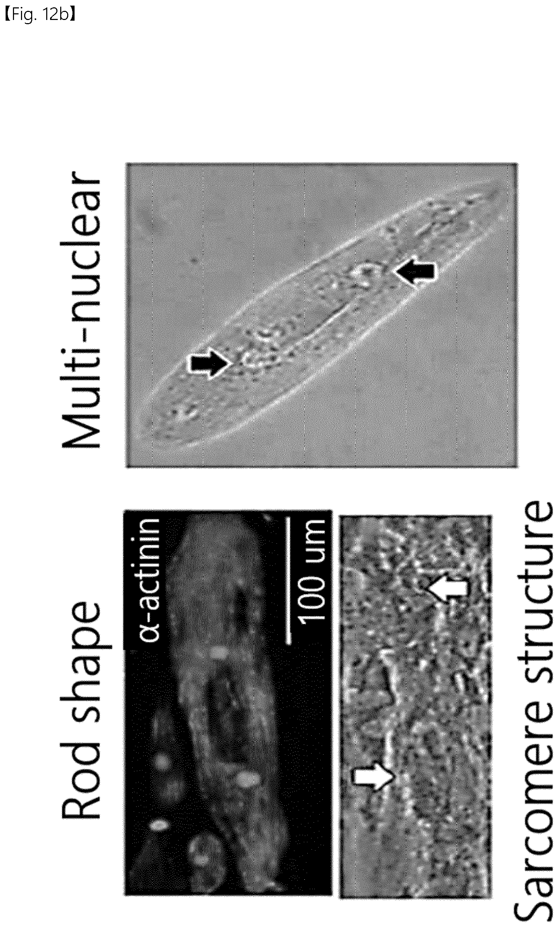

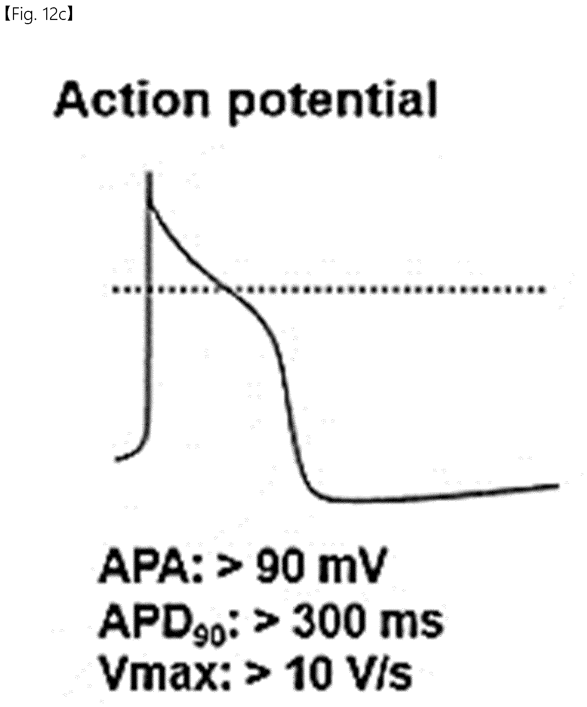

As a result, more than 95% of the cardiomyocytes sorted by CD71 were found to express the cardiomyocyte-specific marker cTnT even after being maintained in vitro for 70 days. Sarcomere structures and multi-nuclear structures showed morphologies of rod-shape cardiomyocytes. The cells exhibit a potential function similar to that of mature cardiomyocytes. Accordingly, it was observed that the maturation of genotype and phenotype in the cells sorted by CD71 was possible even after in-vitro cultivation (FIGS. 12a to 12c).

Therefore, the data implies that CD71 can be used as a surface marker for differentiated contracting cardiomyocytes and cardiomyocytes sorted thereby can maintain characteristics of contracting cardiomyocytes even after in-vitro cultivation for a long time.

Consequently, the present inventors confirmed that CD71 can be used as a surface marker to select homogenous contracting cardiomyocytes of high purity, thereby forming clusters for filtration and transplantation of cardiomyocytes.

### EXAMPLE 4: CD71 Marker on Human induced Pluripotent Stem Cell (hiPSC)-Derived Cardiomyocytes

Characterization of CD71 surface marker on human induced pluripotent stem cell (hiPSC)-derived cardiomyocytes was performed in the same manner as in Examples 1 to 3.

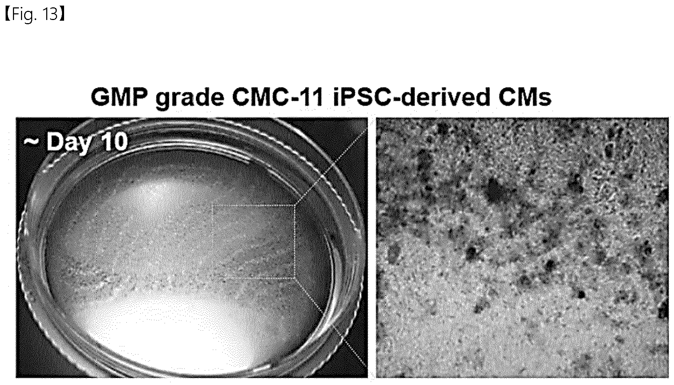

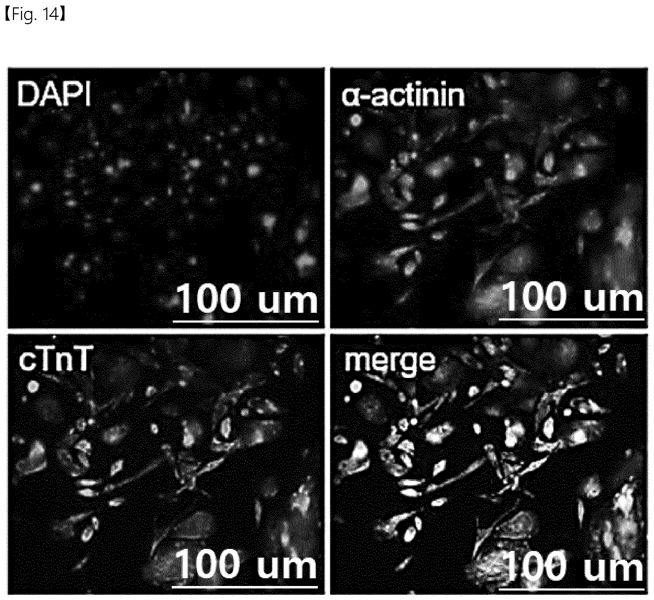

Clinical-grade CMC-11 iPSC cell line 17 was induced to differentiate according to the protocol described in FIG. 1, and contracting cardiomyocytes were observed as in hESC 10 days after initial differentiation (FIG. 13). Immunohistochemical staining identified that the contracting cardiomyocytes sorted by flow cytometry using CD71 surface marker expressed high levels of cTnT and $\alpha$-actinin, which are markers specific for cardiomyocytes (FIG. 14).

Hence, in case for hiPSC-derived cardiomyocytes like hESC-derived cardiomyocytes, CD71 was also observed to serve as a surface marker for contracting cardiomyocytes.

## EXAMPLE 5: Improvement of Cardiomyocyte Cluster in Cell Viability

### 5-1. Improvement in cell viability under hypoxia

In order to form cell clusters, cardiomyocytes ($1 \times 10^5$ cells/ml) selected by CD71 surface marker were transferred to an ultra-low attachment (ULA) culture plate (Corning, Lowell, MA) or a 200-$\mu$m spheroid forming dish (SFD) (SPL life science, Korea) and suspended and incubated in 10 ml of low-glucose DMEM (Gibco) supplemented with 5 % FBS (Gibco).

Cell viability in a hypoxic condition was analyzed. In this regard, single or aggregated cells were cultured in suspension for 3 days in a 3 % $O_2$ incubator (Biofree, Korea). The cultured cells were stained with LIVE/DEAD staining solution (LIVE/DEAD viability kit, Invitrogen) according to the manufacturer's instruction. Fluorescence images were obtained by a florescence microscope (Nikon ECLIS Ti2-U, Nikon, Japan). Total numbers of viable cells (green) and dead cells (blur) were counted in 25 random fields per sample (N = 5).

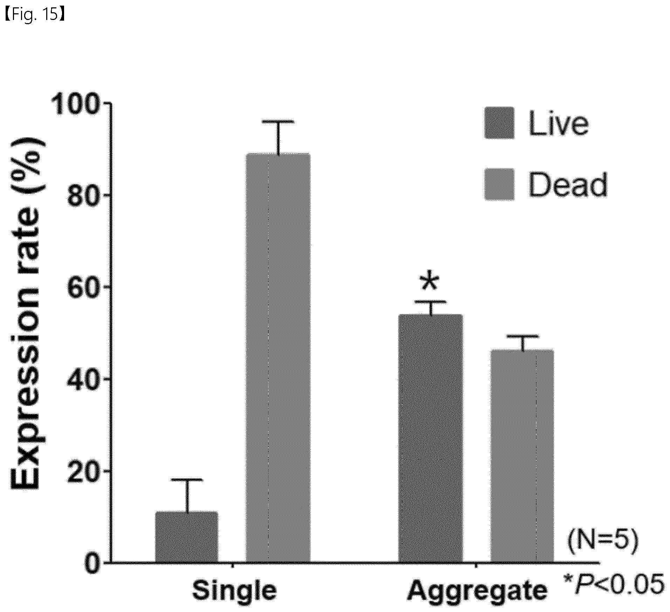

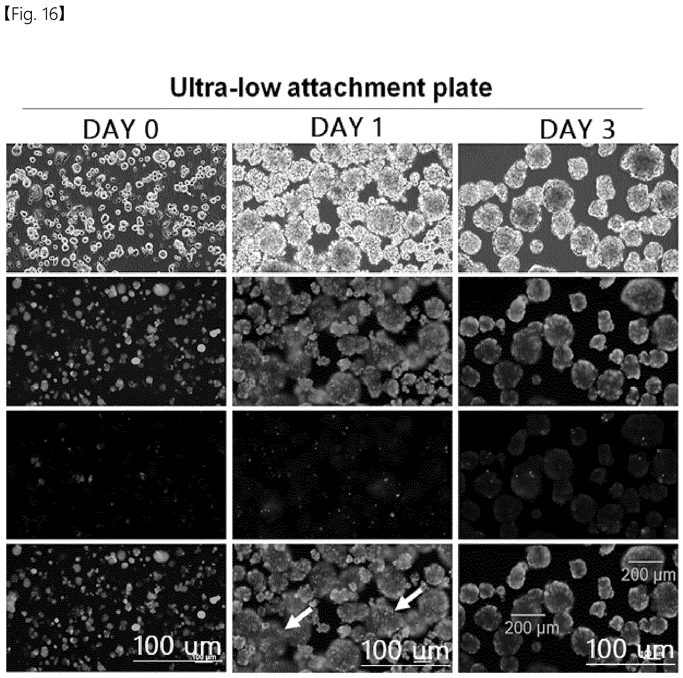

In the viability assay, 90% or more of the single cells were dead while clustered cardiomyocytes were still alive on day 3 (Table 2 and FIG. 15). In addition, when large clusters (diameter > 300 $\mu$m) were formed on day 1, the death rate was high (FIG. 16, white arrow). In contrast, clusters with a diameter less than 200 $\mu$m (FIG. 16, yellow arrow) were observed to survive for 7 days or longer (data not shown).

TABLE 2

|  | Live (%) | Dead (%) |
|---|---|---|
| Single cells | 12-18 | 88-97 |
| Clusters | 55-58 | 45-50 |

### 5-2. Improvement in cell viability after cryopreservation

For cryopreservation, the clusters were gently washed with PBS and resuspended in a culture medium. After centrifugation, the supernatant was removed. The pellet was mixed with CryoStor (CS10; Biolife solution) in Cryo-vial (Cryotube; Thermo Scientific) and frozen at -30°C for 1 hr. Subsequently, the vial was transferred into a LN2 tank (MVE-XC-47/11, Chart-MVE) and cryopreserved for 6 months. After cryopreservation, the frozen vial was warmed in 37°C bead-bath for 1 min, resuspended in 10 ml low-glucose DMEM (Gibco) supplemented with 5 % FBS (Gibco), and centrifuged at 1000 RPM for 3 min. After thawing, cell viability was analyzed with the LIVE/DEAD kit as in Example 5-1.

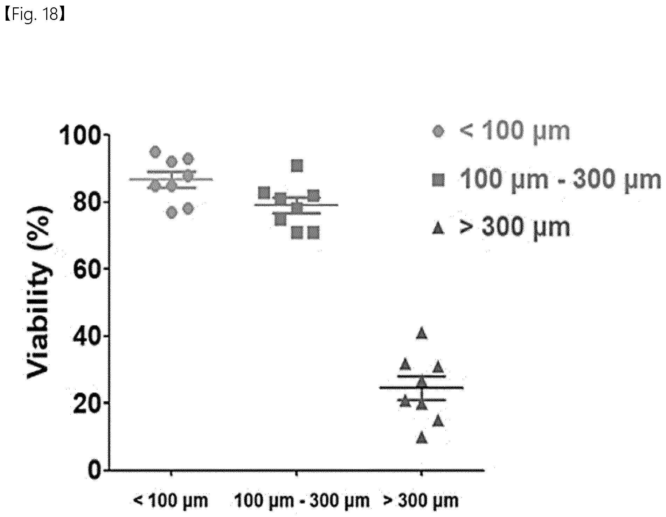

Clusters with a diameter of 300 $\mu$m or less did not greatly decrease in viability (FIGS. 17a to 17c and 18) whereas a significant reduction in viability was observed for clusters with a diameter of 300 $\mu$m or larger after cryopreservation for 6 months (Table 3 and FIG. 18).

TABLE 3

|  | Viability (%) |
|---|---|
| Less than 100 $\mu$m | 78-92 |
| 100 to 300 $\mu$m | 72-90 |
| 300 $\mu$m or larger | 10-40 |

### 5-3. Size optimization of cardiomyocyte cluster

From data of the experiments, it was considered that clusters with diameters less than 200 $\mu$m were optimal in light of the hypoxic and post-cryopreservation viability.

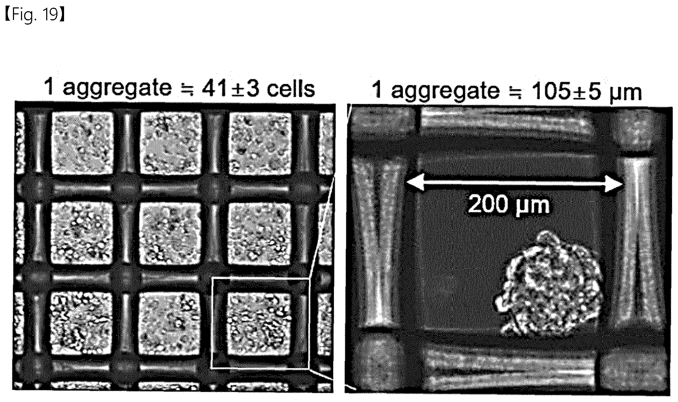

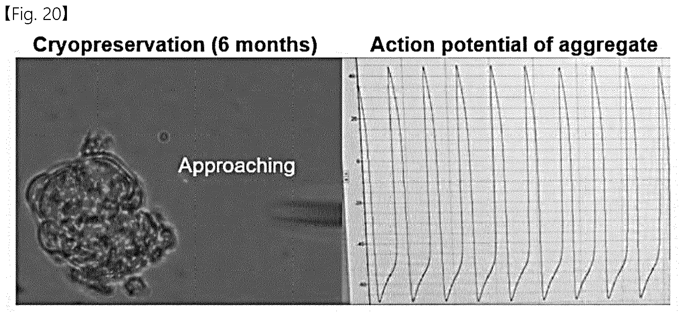

After 38 to 44 single contracting cardiomyocytes were spread in 200-$\mu$m SFD (SPL life science), the cardiomyocytes were cultured to form uniform clusters with a size of 105 $\pm$ 5 $\mu$m (FIG. 19). The uniformly formed clusters were observed to continuously contract for 30 days or longer and survive at 80% or higher even after long-term cryopreservation. In addition, the clusters also retained electrophysiological function (FIG. 20).

The experimental results showed that clusters too small in size decreased in viability in a hypoxic condition and the viability of clusters as large as or larger than 200 $\mu$m drastically dropped upon cryopreservation. As opposed, clusters with a size of 105 $\pm$ 5 $\mu$m were highly viable even after long-term cryopreservation and the cardiomyocytes were observed to continuously contract. In view of the hypoxia and cryopreservation, 105 $\pm$ 5 $\mu$m was considered to be an optimal size for the clusters.

**EXAMPLE 6: Therapeutic Efficacy of Cardiomyocyte Cluster for Acute Myocardial Infarction**

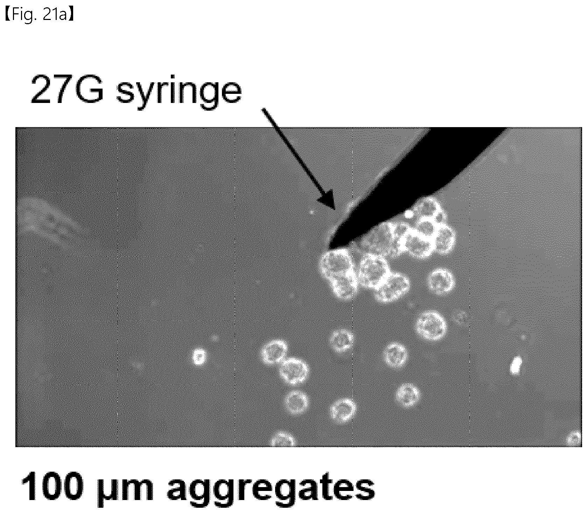

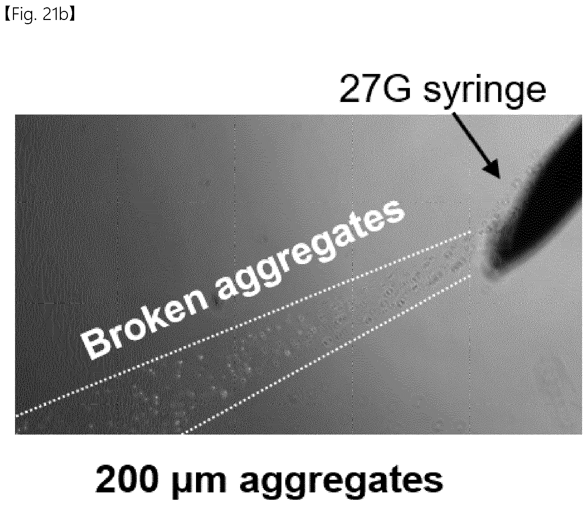

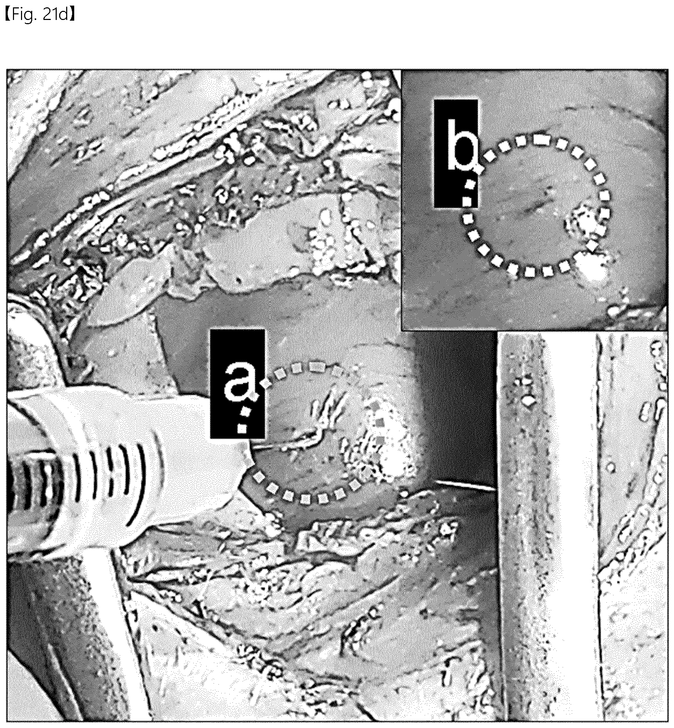

The therapeutic efficacy of cardiomyocyte clusters was analyzed by evaluating the effect of improving heart function when the cardiomyocyte clusters were administered in vivo. Prior to experiments in rats, a syringe with 27 G needle was tested for injecting cardiomyocyte clusters into the body. Clusters with a size of 100 $\mu$m were not morphologically changed while passing through 27G syringes (FIG. 21a) wherein the clusters 200 $\mu$m in size were ruptured during the passage through 27G syringes (FIG. 21b). The test result indicates that clusters with a size less than 200 $\mu$m can be applied to the catheter system.

**6-1. Recovery effect of cardiomyocyte cluster on damage heart function**

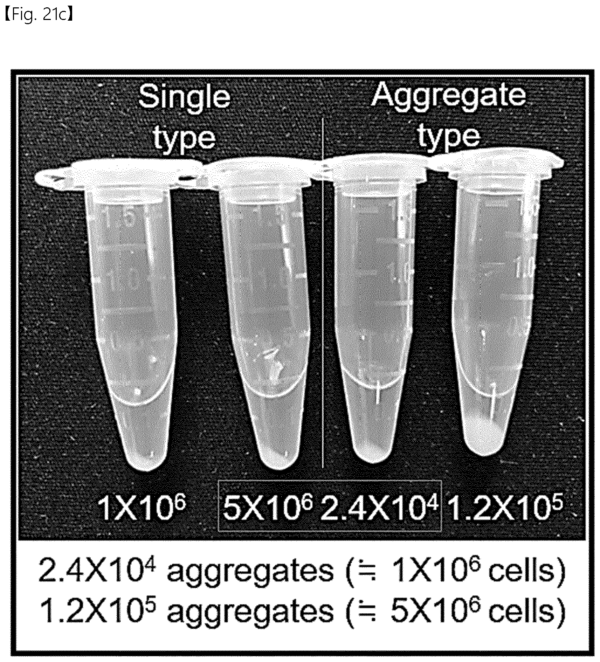

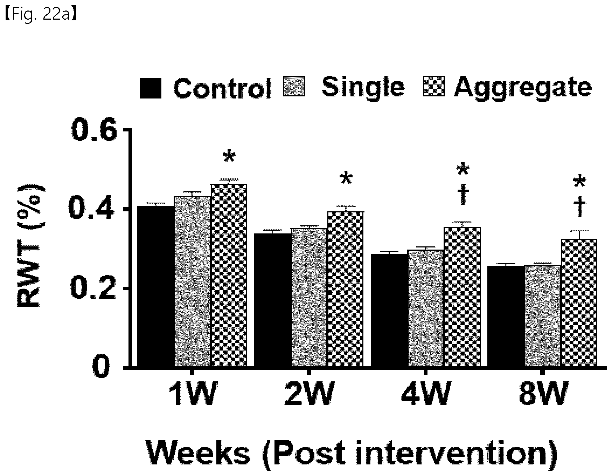

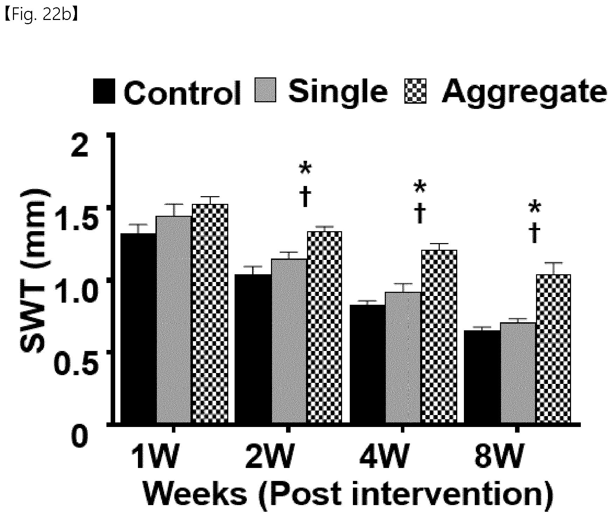

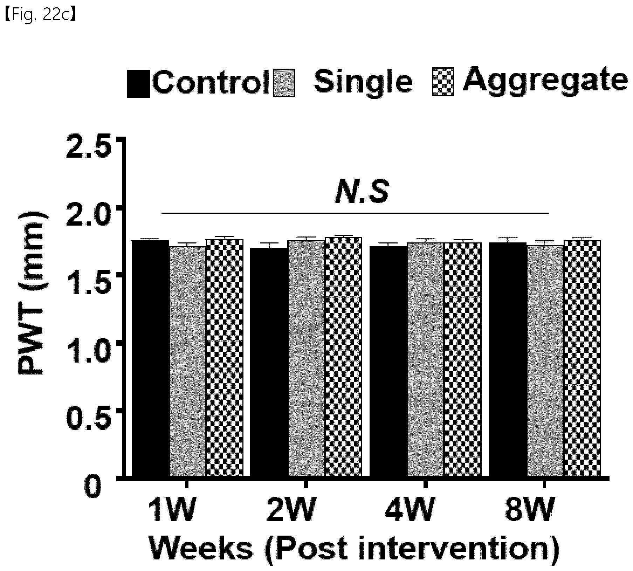

Fisher 344 rats (160-180g, 8 weeks old, male, Koatec, Korea) were anesthetized with 2 % inhaled isoflurane and intubated via the trachea with an intravenous catheter. Myocardial infarction (MI) was achieved by tying a suture with sterile polyethylene glycol tubing placed into the left anterior descending coronary artery (LAD) for 1 min, and then the knot was permanently ligated using a 7-0 Prolene suture to establish a myocardial infarction group (hereinafter referred to as "MI group"). Immediately after the LAD occlusion, PBS, single cardiomyocytes, or the cluster formed above, wherein the cardiomyocytes were labeled with chloromethylbenzamido (CellTracker CM-DiI), was injected into two different sites on the border zone of the infarcted myocardium (FIGS. 21c to 21d). According to the injected substances, the rats were divided into a control (PBS administered), a single cell-administered group (single cardiomyocytes $5 \times 10^6$), and a cluster-administered group (105 $\pm$ 5 $\mu$m cardiomyocyte cluster $2 \times 10^4$). Subsequently, echocardiography was performed using a transthoracic echocardiography system equipped with a 15-MHz L15-7io linear transducer (Affniti 50G, Philips), and echocardiograms were recorded 1, 2, 4, and 8 weeks after the patch attachment. Left-ventricular end-diastolic diameter (LVIDd), left-ventricular end systolic diameter (LVISd), related wall thickness (RWT), septal wall thickness (SWT), and posterior wall thickness (PWT) were measured (FIGS. 22a and 22c), and ejection fraction (EF) and fractional shortening (FS) were calculated with the following equations:

$$EF\ (\%)\ =\ [(LVEDV-LVESV)/LVEDV]\ *\ 100$$

$$FS\ (\%)\ =\ [(LVEDD-LVESD)/LVEDD]\ *\ 100$$

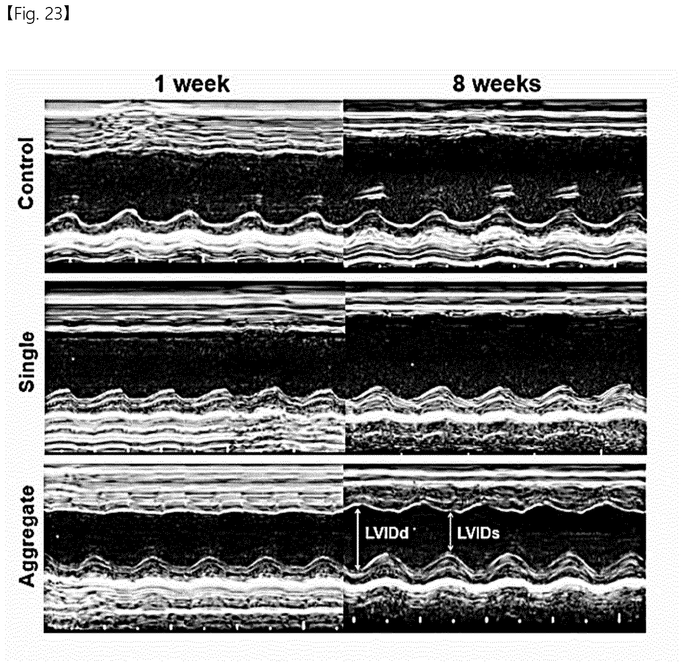

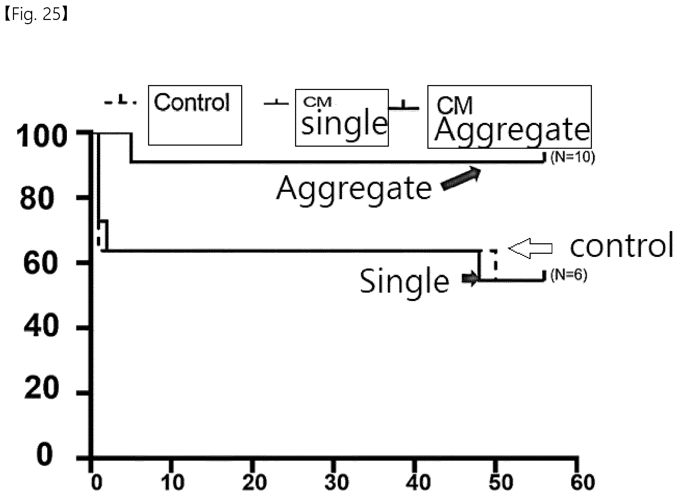

The cluster-administered group was measured to remarkably decrease in left-ventricular end-diastolic diameter (LVIDd) and left-ventricular end systolic diameter (LVISd), compared to the control and the single cell-administered group, after 8 weeks (Table 4 and FIGS 23 and 25).

TABLE 4

|  | LVIDd (mm) | | | LVIDs (mm) | | |
|---|---|---|---|---|---|---|
|  | Control | Single cell administered | Cluster administered | Control | Single cell administered | Cluster administered |
| 1 Week | 7.57 | 7.31 | 7.06 | 6.26 | 6.01 | 5.66 |
| 2 Weeks | 8.10 | 8.21 | 7.85 | 6.78 | 6.83 | 6.38 |
| 4 Weeks | 8.79 | 8.92 | 8.23 | 7.53 | 7.57 | 6.71 |
| 8 Weeks | 9.29 | 9.35 | 8.62 | 8.09 | 8.03 | 7.04 |

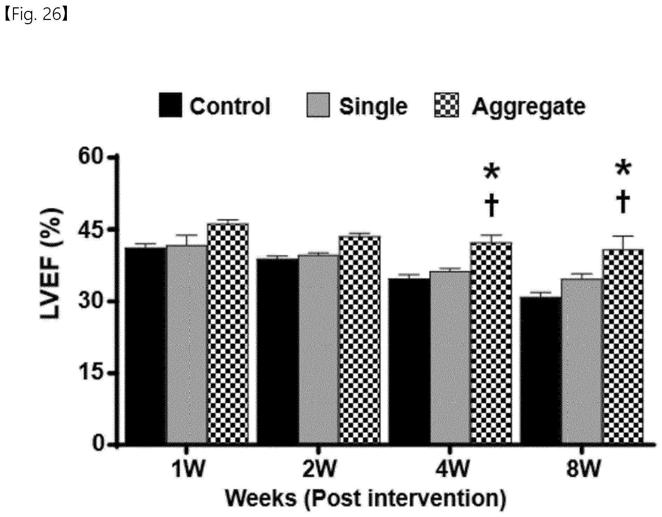

Particularly, fraction (LVEF) and fractional shortening (FS), which are important indices for assessing cardiac function, were remarkably improved in the cluster-administered group, compared to the control and the single cell-administered group (Table 5 and FIGS. 26 to 27).

TABLE 5

|  | LVEF (%) | | |
| --- | --- | --- | --- |
|  | Control | Single cell administered | Cluster administered |
| 1 Week | 40.9 | 41.7 | 45.8 |
| 2 Weeks | 38.7 | 39.7 | 43.9 |
| 4 Weeks | 34.6 | 36.3 | 42.8 |
| 8 Weeks | 31.4 | 34.6 | 42.4 |

TABLE 6

|  | FS (%) | | |
| --- | --- | --- | --- |
|  | Control | Single cell administered | Cluster administered |
| 1 Week | 17.3 | 17.7 | 19.7 |
| 2 Weeks | 16.2 | 16.7 | 18.8 |
| 4 Weeks | 14.3 | 15.1 | 18.3 |
| 8 Weeks | 12.9 | 14.4 | 18.3 |

The data obtained above demonstrate that when the transplantation of the cardiomyocyte cluster according to the present invention is more effective for recovery from damaged cardiac function, compared to single cardiomyocyte transplantation.

**6-2. Hemodynamic assessment for cardiac function improvement of cardiomyocyte cluster**

Hemodynamic assessment was conducted on the MI-induced rats in the control, the single cell-administered group, and the cluster-administered group of Example 6-1.

PBS, single cardiomyocytes, and cardiomyocyte cluster were injected to induced myocardial infarction as in Example 6-1. Hemodynamic analysis was performed 8 weeks before euthanasia. After thoracotomy, the left ventricle of the heart was perforated using a 26-gauge needle and an SF conductance catheter (SPR-838, Millar) was inserted to the left ventricle. The left ventricular pressure-volume (PV) parameters were continuously recorded using a PV conductance system (MPVS Ultra, emka TECHNOLOGIES, France) coupled to a digital converter (PowerLab 16/35, ADInstruments, Colorado Springs, USA). In order to additionally examine a load-independent cardiac function, slopes of ESPVR (end-systolic pressure-volume relationship) and EDPVR (end-diastolic pressure volume relationship) were analyzed during transient inferior vena cava (IVC) occlusion.

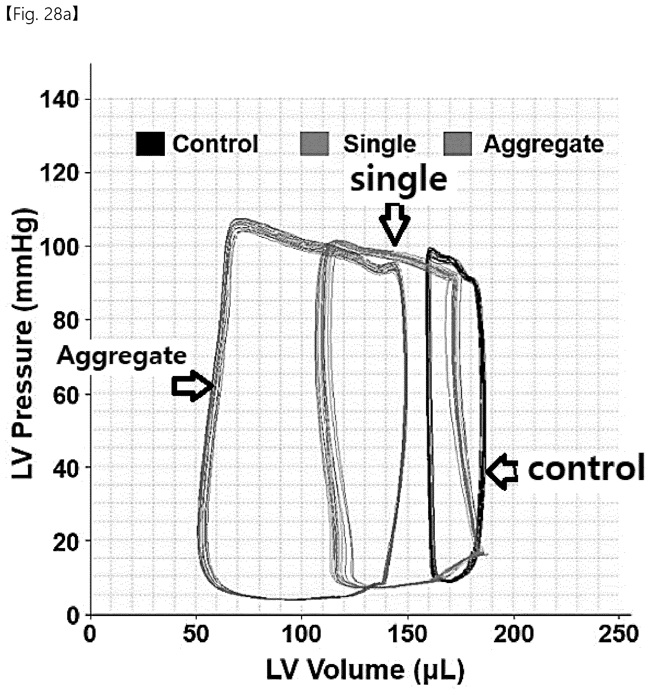

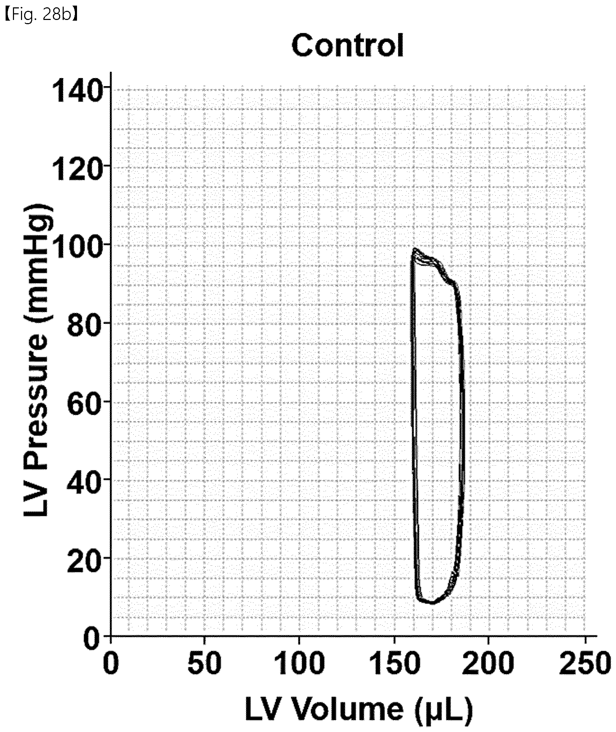

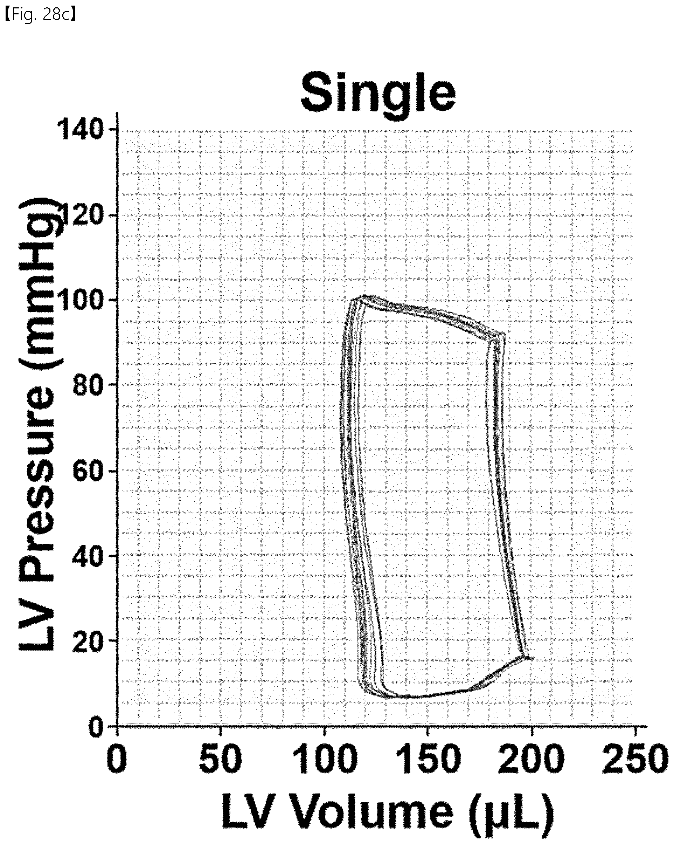

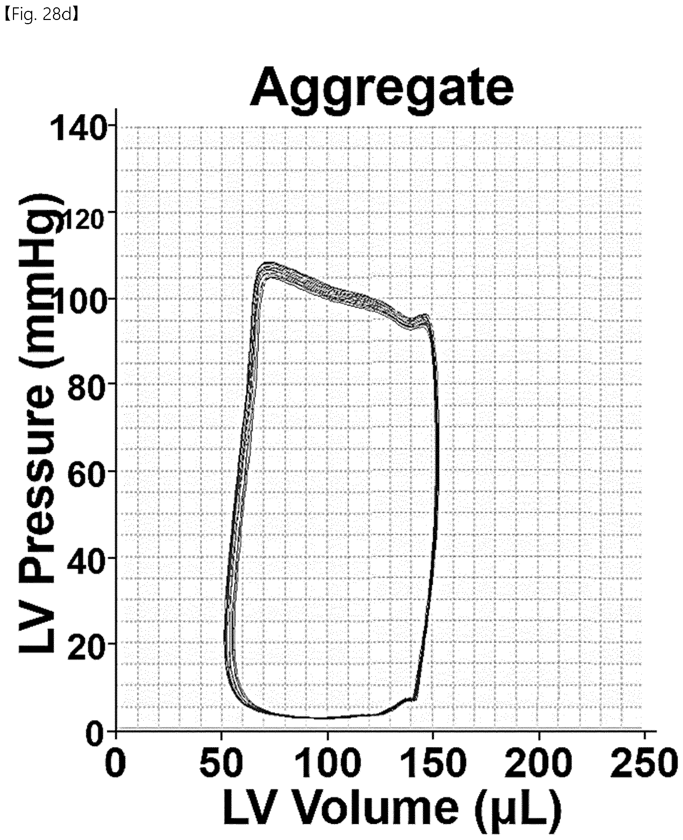

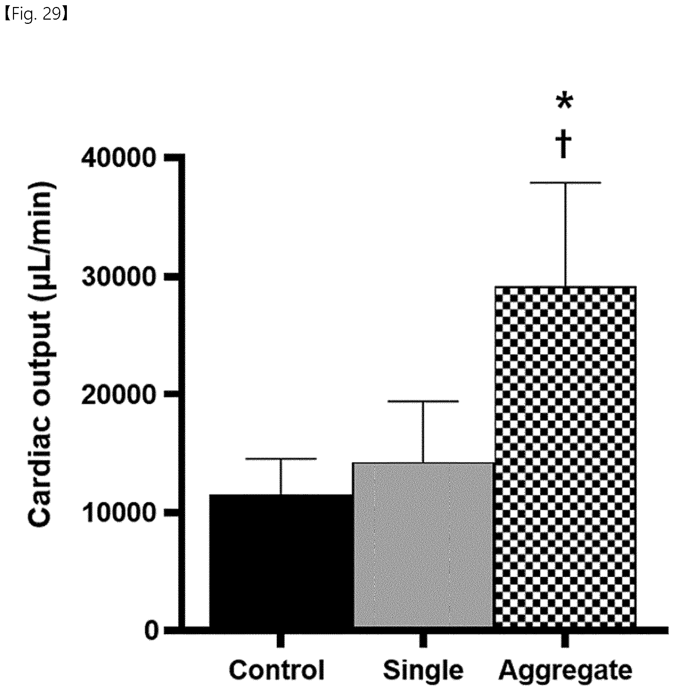

As a result of the hemodynamic assessment, pressure-volume loops for the left ventricle are depicted in FIGS. 28a to 28d. The cluster group was observed to increase in the hemodynamic indices such as cardiac output (CO) and stroke volume (Tables 7 to 8 and FIGS. 29 to 30).

TABLE 7

|  | Control | Singe cell administered | Cluster administered |
| --- | --- | --- | --- |
| Cardiac output ($\mu$L/min) | 11522 | 14243 | 29200 |

TABLE 8

|  | Control | Singe cell administered | Cluster administered |
| --- | --- | --- | --- |
| Stroke volume ($\mu$L) | 33.004 | 42.586 | 82.601 |

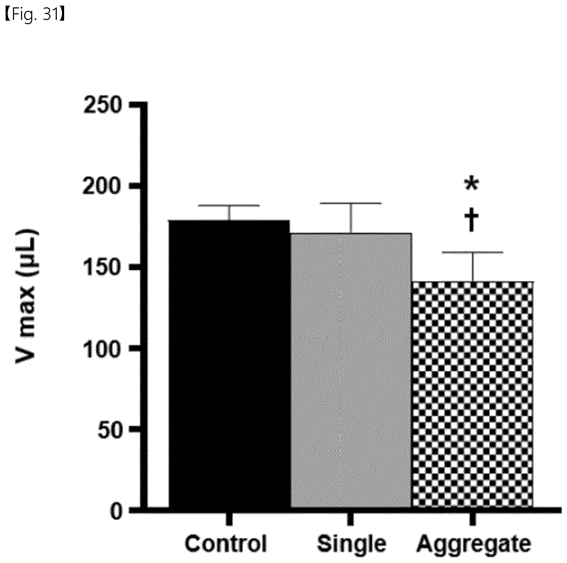

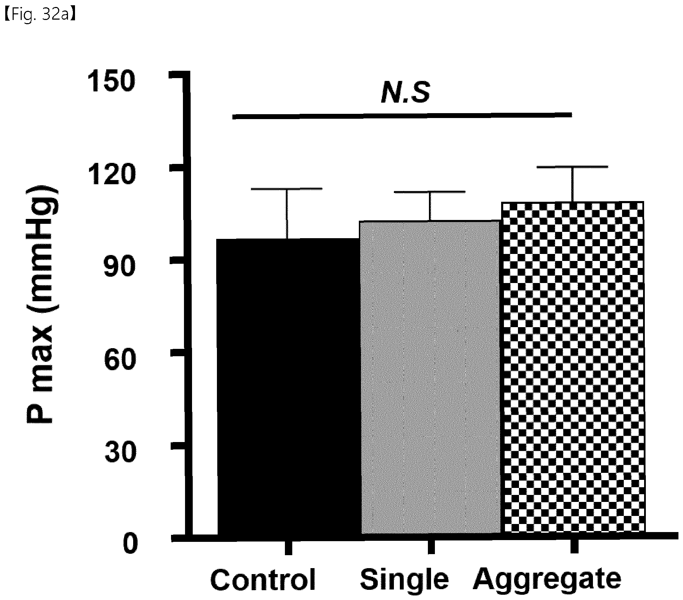

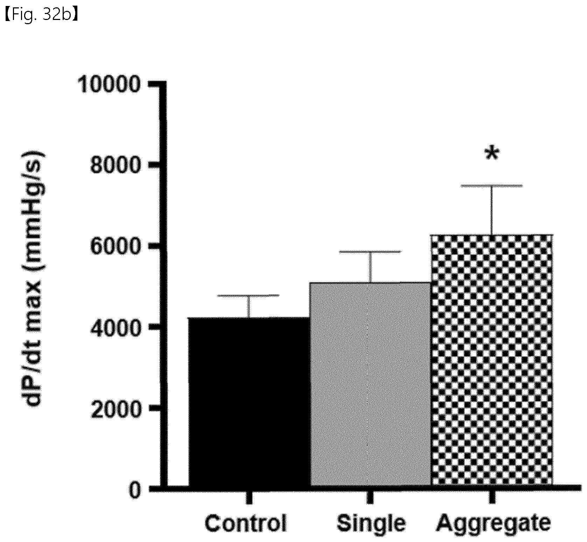

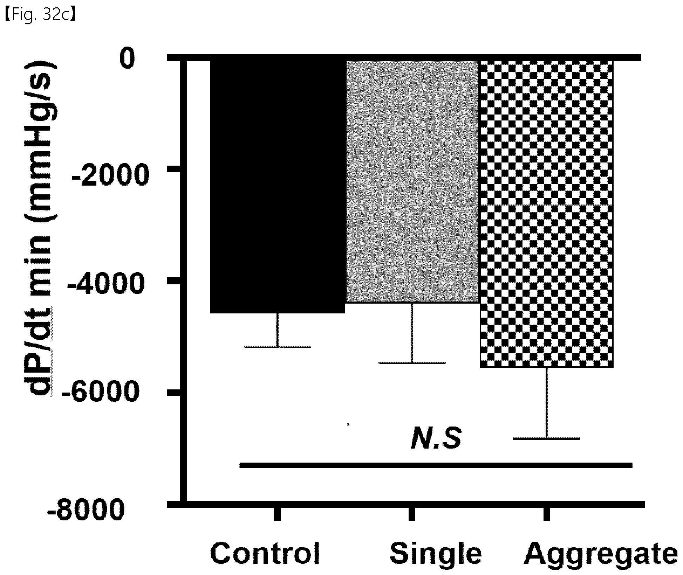

In addition, the cluster group was lower in the maximum volume (Vmax), which is associated with negative cardiac remodeling (Table 9 and FIG. 31) and higher in the maximum pressure (Pmax) at the maximum systole than the other two groups. Furthermore, a higher maximum pressure change was detected in the cluster group as the dP/dt max, which is an index accounting for cardiac function improvement, and was about 1.5 fold higher than in the other two groups. In contrast, the cluster group was lower in dP/dt min, which is the minimum pressure change during diastole, than the other two groups (Table 10 and FIGS. 32a to 32c).

TABLE 9

|  | Control | Singe cell administered | Cluster administered |
|---|---|---|---|
| V max ($\mu$L) | 178.92 | 171.54 | 141.56 |

TABLE 10

|  | Control | Singe cell administered | Cluster administered |
|---|---|---|---|
| P max (mmHg/s) | 96.778 | 102.482 | 108.258 |
| dP/dt max (mmHg/s) | 4239.6 | 5096.8 | 6268.5 |
| dP/dt min (mmHg/s) | -4578.6 | -4403.4 | -5552.67 |

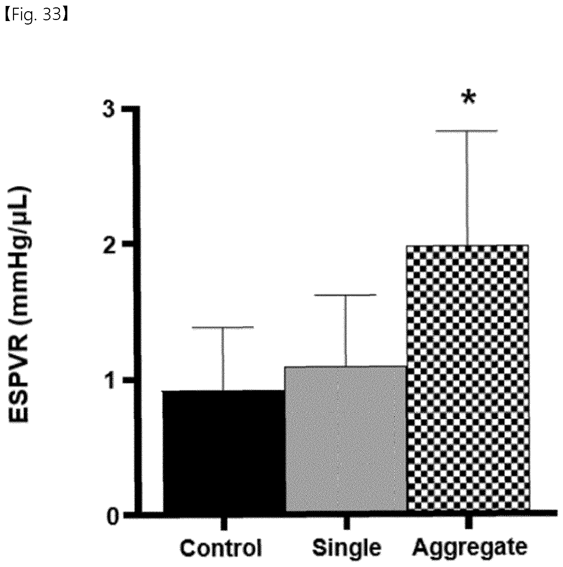

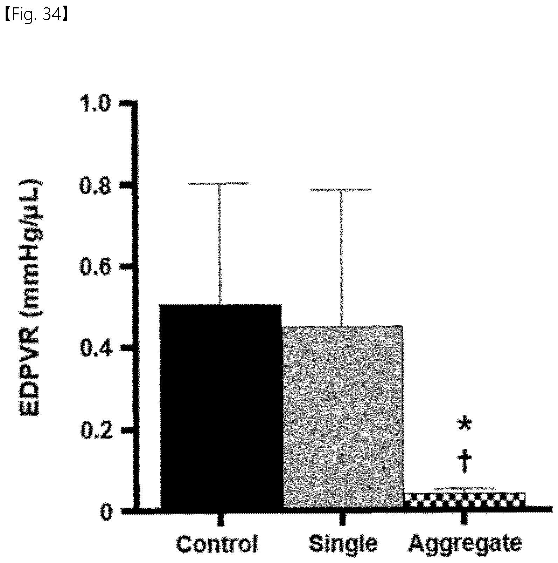

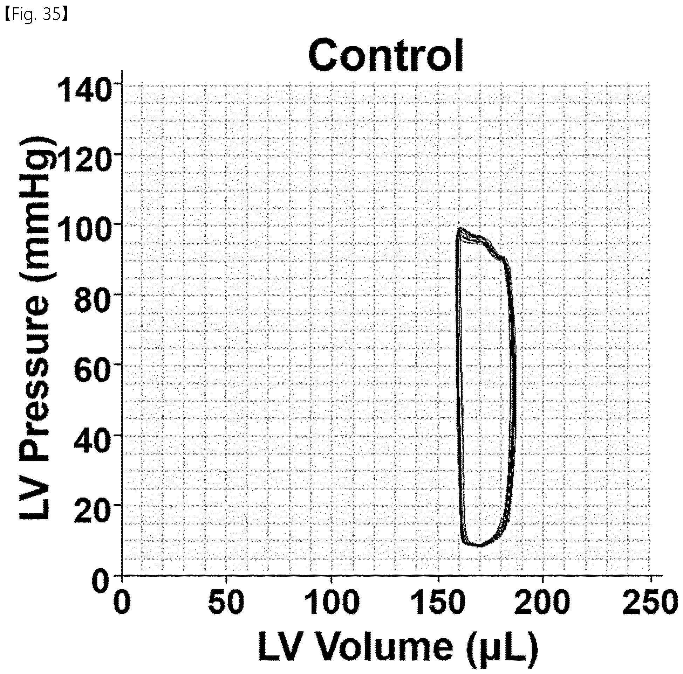

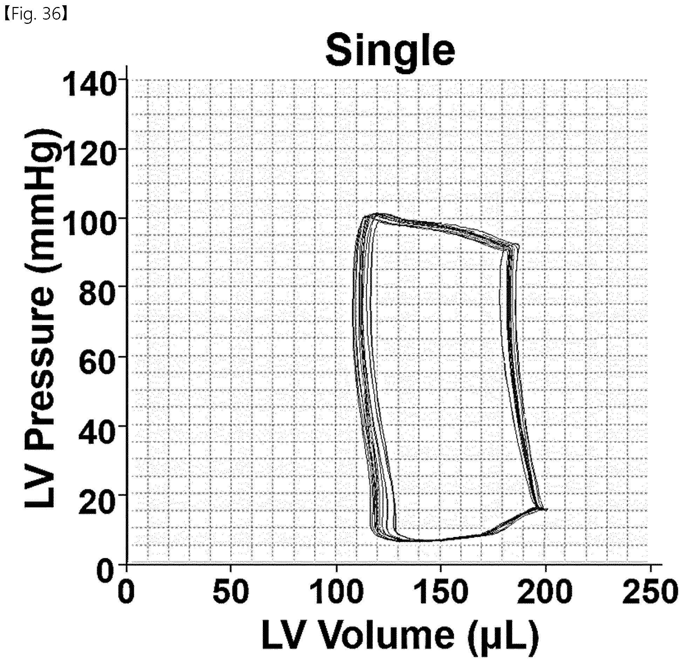

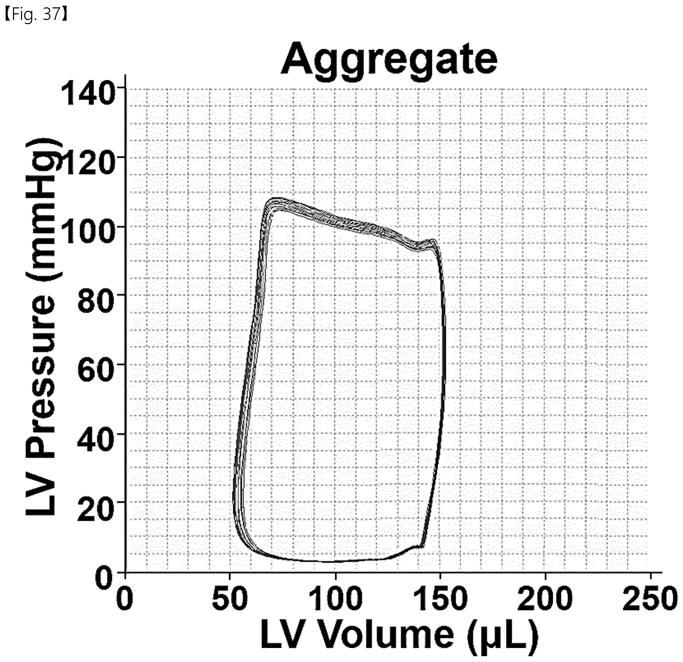

The cluster group was measured to be higher in ESPVR and lower in EDPVR than the other two groups (Tables 11 to 12 and FIGS. 33 to 37), showing an increase in load-independent cardiac contractility after myocardial infarction.

TABLE 11

|  | Control | Singe cell administered | Cluster administered |
|---|---|---|---|
| ESPVR (mmHg/$\mu$L) | 0.9382 | 1.0937 | 1.9835 |

TABLE 12

|  | Control | Singe cell administered | Cluster administered |
|---|---|---|---|
| EDPVR (mmHg/$\mu$L) | 0.37339 | 0.45271 | 0.04112 |

Comprehensively, the hemodynamic assessment data were coincident with the ultrasonography of Example 61, implying that the cell cluster according to the present invention can significantly improve the cardiac function of the subject transplanted therewith.

**EXAMPLE 7: Cell Viability after Cardiomyocyte Cluster Injection**

To assess the distribution and viability of cardiomyocytes injected into a myocardial infarction zone of a transplanted subject, immunohistochemistry was performed.

PBS (control), single cardiomyocytes (single cardiomyocytes $5 \times 10^6$ administered; single cell-administered group), and cardiomyocyte cluster (cardiomyocyte cluster $2 \times 10^4$ administered; cluster-administered group) were injected into infarcted myocardium before cardioectomy. The isolated hearts were frozen. Here, the cardiomyocytes were labeled with red fluorescent dye Dil before injection. The frozen cardiac tissue was washed with PBS, permeabilized with 0.5 % Triton X-100 in PBS for 15 min, and blocked in 3 % BSA at room temperature for 1 hr, followed by incubation overnight at 4°C with primary antibodies such as ACTN2 (Sigma), cTnT (ThermoFisher), MYH6/7 (Abcam), hMYH7 (Abcam), CD31 (Abcam), and GJA1 (Abcam). The cardiac tissue was washed three times with PBS and incubated with a secondary antibody at room temperature for 1 hr. As the secondary antibody, anti-mouse/rabbit IgG Alexa Fluor 488 (Invitrogen) or anti-mouse/rabbit IgG Alexa Fluor 647 (Invitrogen) was used. Immunohistochemistry assay was performed for 3 to 5 independent samples and all images were acquired and analyzed using Nikon AIR HD25 confocal microscope (Nikon Corp, Japan). To quantitate the viability of the transplanted cells, densities of cTnT expressing-Dil labeled cells (Dil+/cTnT+) were calculated in the upper, medium, and lower parts of the left ventricular tissue. TUNEL assay was performed with the cardiomyocyte-specific marker MYH6/7 to quantitate the ratio of dead cardiomyocytes in the transplanted cardiomyocytes.

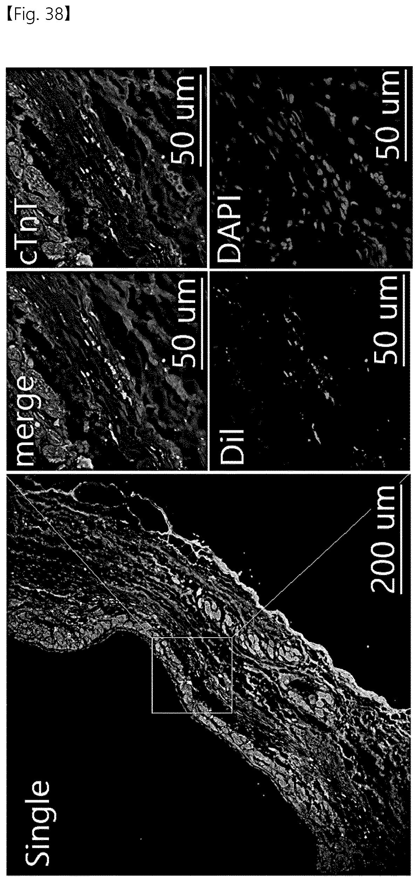

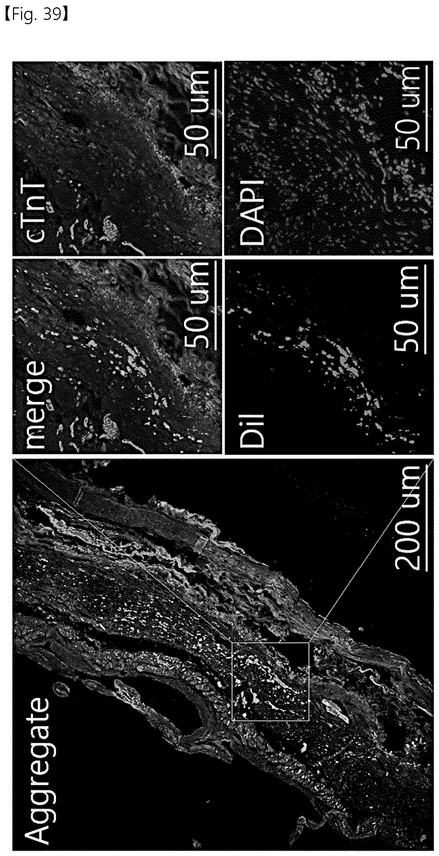

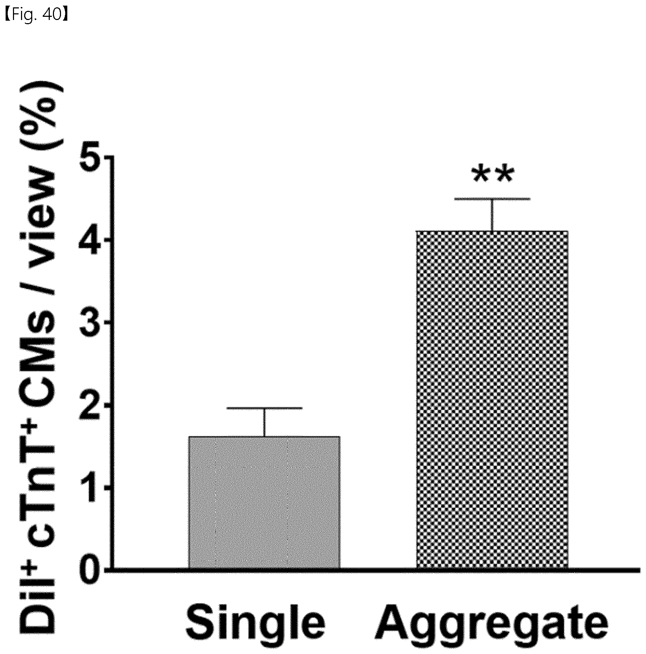

The assay results showed that cardiomyocytes were distributed in a wide area of the left ventricle in the cluster-administered group, but found in limited locations in the single cell-administered group (FIGS. 38 and 39). In addition, the viability of DiI+/cTnT+ cells increased by about three fold in the cluster-administered group than the single cell-administered group, as measured by quantitation analysis (Table 13 and FIG. 40).

TABLE 13

|  | Singe cell administered | Cluster administered |
|---|---|---|
| DiI+ cTnT+ CMs/view (%) | 1.6 | 4.2 |

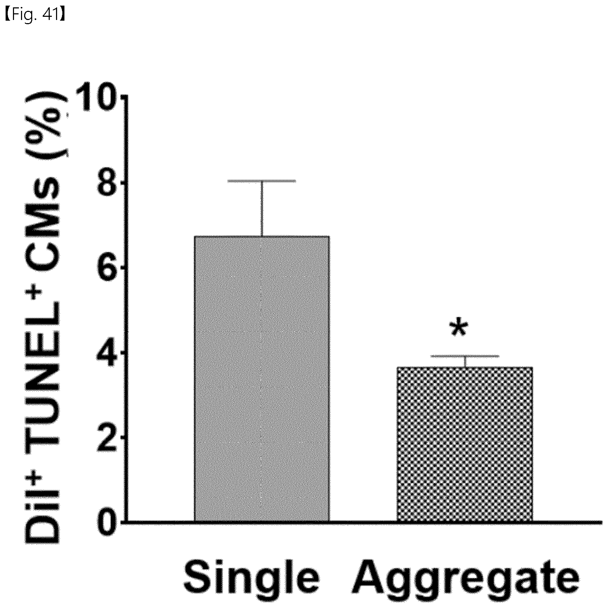

DiI+/TUNEL+ cardiomyocytes were detected at a remarkably low ratio in the cluster-administered group as measured by the TUNEL assay, thus accounting for very low ratio of dead cells among the cardiomyocytes transplanted into the infarcted myocardium (Table 14 and FIG. 41).

TABLE 14

|  | Singe cell administered | Cluster administered |
|---|---|---|
| DiI+ TUNEL+ CMs (%) | 6.8 % | 3.7 % |

The result shows that the cardiomyocyte cluster according to the present invention increases the viability of individual cardiomyocytes upon transplantation into a subject, implying that the cardiomyocyte cluster according to the present invention makes a great contribution to the increase of cell viability in light of the fact that the number of cardiomyocytes transplanted in the cluster-administered group ($2 \times 10^4$ cells) was significantly lower than that in the single cell-administered group ($5 \times 10^6$ cells).

**EXAMPLE 8: Improving Effect of Cardiomyocyte Cluster on Cardiomyocyte Engraftment**

The cardiac tissue frozen as in Example 7 was analyzed by immunohistochemistry using ACTN2 (Sigma), cTnT (ThermoFisher), MYH6/7 (Abcam), hMYH7 (Abcam), CD31 (Abcam), and GJA1 (Abcam) as primary antibodies and anti-mouse/rabbit IgG Alexa Fluor 488 (Invitrogen) or anti-mouse/rabbit IgG Alexa Fluor 647 (Invitrogen) as a second antibody.

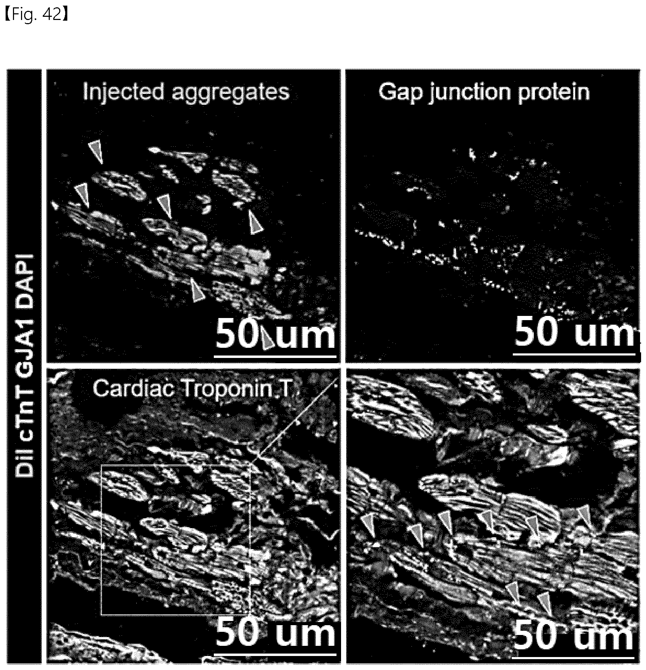

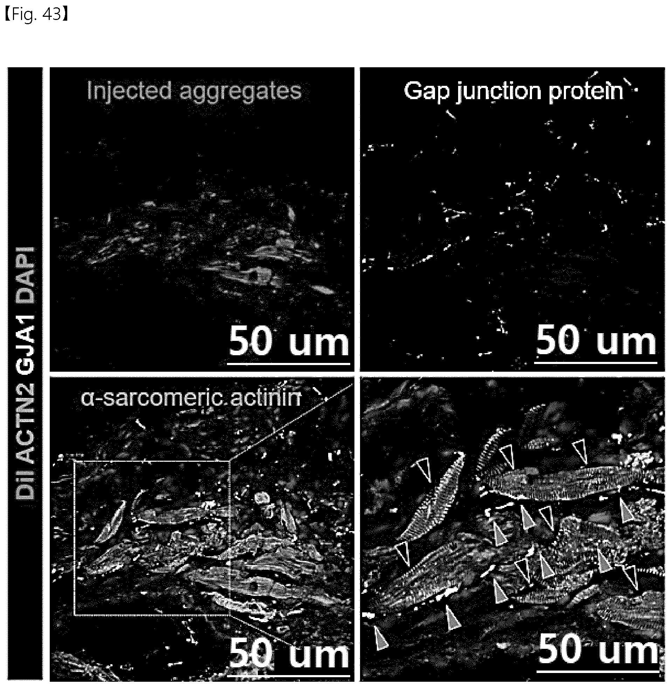

As a staining result, the cardiomyocytes in the cluster group were observed to appear in a rod shape similar to that of mature cardiomyocytes, with the expression of gap junction alpha-1 protein (GJA1) detected between cTnT-expressing cells (FIG. 42). In addition, a sarcomere structure (ACTN2) was distinctively detected, with a gap junction formed with neighboring cells. These patterns were observed only in the cluster group, but not in the single cell group (FIG. 43).

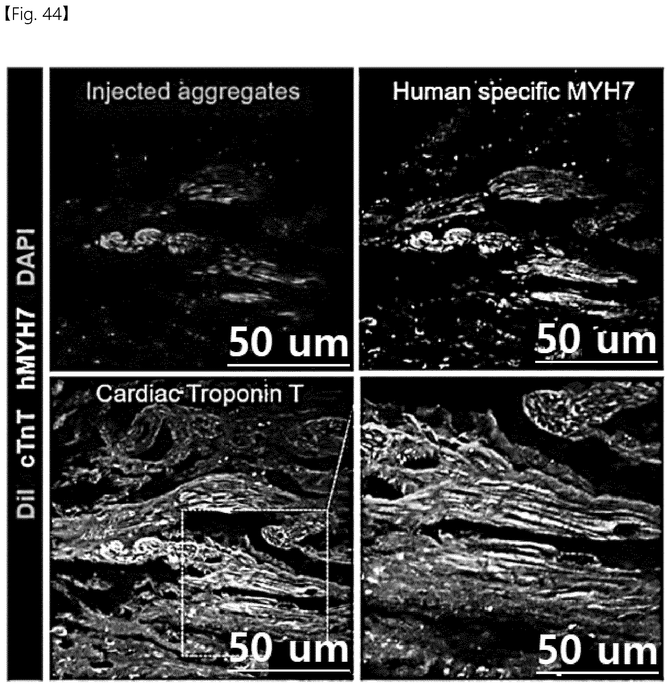

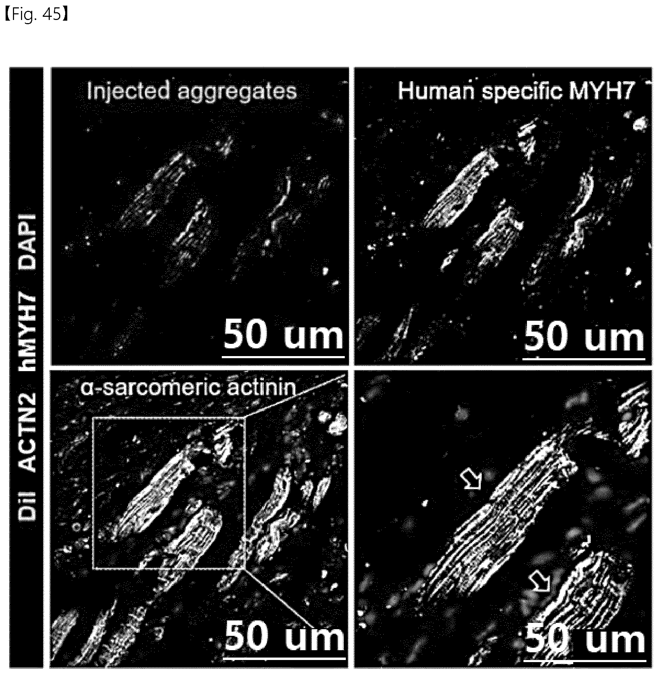

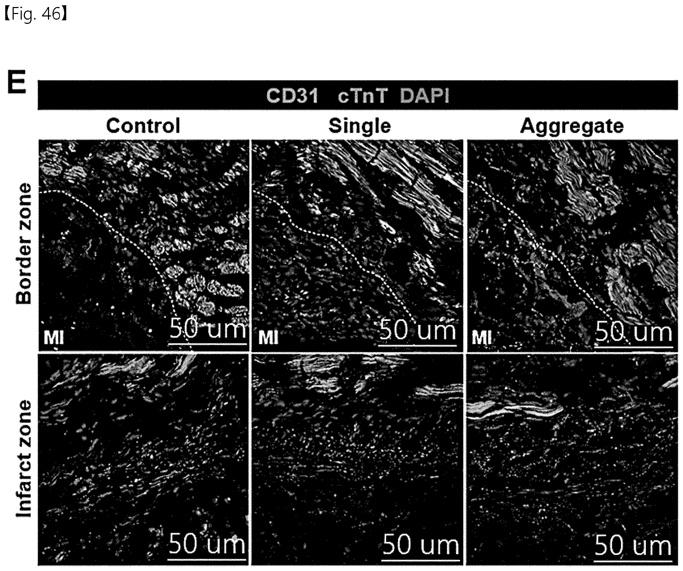

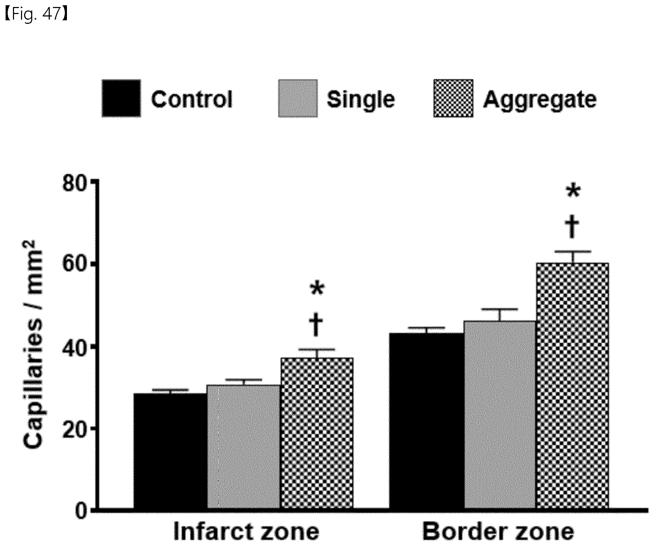

Moreover, DiI-expressing cells were exactly coincident with hMYH7 expression, and rectangular cells similar to adult cardiomyocytes were also observed (FIGS. 44 and 45). When measured by immunostaining using CD31 antibody, the capillary density per area was very high in both the infarcted zone and the border zone where cTnT-expressing cells were observed, for the cluster group (FIG. 46). A capillary density of 50% or higher was detected at the border zone and was the highest in the entire population (Table 15 and FIG. 47).

TABLE 15

|  | Control | Singe cell administered | Cluster administered |
|---|---|---|---|
| Infarct Zone (mm$^2$) | 28 | 30 | 38 |
| Border Zone (mm$^2$) | 42 | 44 | 60 |

These results implicate that when transplanted in the form of clusters into a tissue, the cells are induced to grow mature, giving the prediction that cardiomyocytes transplanted in the form of clusters according to the present invention would exhibit an indirect effect of inducing angiogenesis. Consequently, the transplantation of cardiomyocytes via the cluster according to the present invention is expected to allow the stable engraftment of cardiomyocytes and effectuate a therapeutic effect on infarcted heart.

Industrial Applicability

The present invention relates to a fabrication technique for cardiomyocyte cluster with improved viability in a cryopreservation and hypoxic condition. The cardiomyocyte cluster fabricated according to the present invention highly survives hypoxia and can be cryopreserved for a long time. After long-term cryopreservation, the cardiomyocyte cluster exhibits excellent engraftment and cardiac function improvement upon transplantation into the heart.

**Claims**

1. A method for fabrication of a cluster of cardiomyocytes derived from human totipotent stem cells, the method comprising the steps of:

   culturing human totipotent stem cells to differentiate into cardiomyocytes;
   selecting CD71 positive cardiomyocytes, which express the CD71 surface marker, from the differentiated cardiomyocytes, to form a population of specific cardiomyocytes; and
   forming a cluster ranging in diameter from 100 to 300 $\mu$m from the selected specific cardiomyocytes through cultivation.

2. The method of claim 1, further comprising the steps of: freezing the formed cluster for cryopreservation; and thawing the frozen cluster.

3. The method of claim 1, wherein the specific cardiomyocyte population in the selecting step includes CD71-positve cardiomyocytes in an amount of 40% or more.

4. The method of claim 1, wherein the CD71-positve cardiomyocytes in the selecting step are separated by flow cytometry or manually.

5. The method of claim 1, wherein the forming step is carried out by culturing the specific cardiomyocytes in a spheroid forming dish (SFD) having a diameter of 180 to 220 $\mu$m.

6. The method of claim 1, wherein the forming step is carried out by culturing the specific cardiomyocytes to form a cardiomyocyte cluster ranging in diameter from 100 to 110 $\mu$m.

7. The method of claim 1, wherein the forming step is carried out by culturing the specific cardiomyocytes in a spheroid forming dish having a diameter of 190 to 210 $\mu$m to form a cardiomyocyte cluster ranging in diameter from 100 to 110 $\mu$m.

8. The method of claim 2, wherein the cluster in the freezing step is cryopreserved in a 10% DMSO solution for 6 months or longer.

9. The method of claim 2, wherein the thawing step comprises suspending the cluster in a low-glucose DMEM medium.

10. A pharmaceutical composition comprising CD71 surface marker-expressing cardiomyocytes derived from human totipotent stem cells as an active ingredient for alleviation, suppression, prevention, or treatment of a heart disease, wherein the cardiomyocytes in the form of a cardiomyocyte cluster have a diameter of 100 to 300 $\mu$m.

11. The pharmaceutical composition of claim 10, wherein the heart disease is selected from the group consisting of angina pectoris, myocardial infarction, valvular disease, cardiac failure, cardiac hypertrophy, arrhythmia, pericarditis, and endocarditis.

12. The pharmaceutical composition of claim 10, wherein cardiomyocyte cluster comprises CD71 surface marker-expressing cardiomyocytes in an amount of 40% or higher.

13. The pharmaceutical composition of claim 10, wherein the cardiomyocyte cluster ranges in diameter from 100 to 110 $\mu$m.

【Fig. 1】

【Fig. 2】

【Fig. 3】

【Fig. 4】

【Fig. 5a】

| | |
|---|---|
| A | NXK2_5-undiff |
| B | NXK2_5GFP0-B0 |
| C | NXK2_5GFP0-BX |
| D | NKX2_5-FACS |

【Fig. 5b】

【Fig. 5c】

| a | | |
|---|---|---|
| LOC100132057 | LOC101927468 | BOLA2B |
| ZC3H14 | NBPF11 | TKT |
| HIST1H2BC | NBPF8 | ZNF510 |
| OPN1LW | FAM231D | HIST2H3C |
| RASIP1 | LOC388692 | ZDBF2 |
| LOC101927120 | GRHL3 | MAP3K9 |
| NCF1C | NCAPH | BCL2L2-PABPN1 |
| GRAMD2 | SRP72 | CFAP70 |
| ATCAY | HIST1H2AC | LRRK2 |
| ATP5J2-PTCD1 | FRG2 | TRMT112 |
| UTP18 | LILRB3 | GP5 |
| TECR | FCGR1C | CHN1 |
| COA6 | LINC00664 | MAPK7 |
| MSMO1 | FAM90A7P | ATPAF1 |
| LOC283299 | ZNF740 | KIF16B |
| LINC01521 | RNU1-13P | HHIPL2 |
| CD24 | POU5F1 | TMPRSS11F |
| HDGFRP2 | WASH5P | SLCO1C1 |
| KLK4 | CEP63 | SNAPC4 |
| XAGE1E | RAB5A | KCNT2 |
| FGFBP3 | CCT7 | C5orf42 |
| LY6G5C | SLC48A1 | RER1 |
| XAGE1B | WBSCR16 | LOC642852 |
| PLEKHG5 | RNF4 | EPB41L5 |
| MGST1 | ALKBH1 | PCDHGA3 |
| PET100 | TATDN2 | |
| PCBD2 | ACLY | |

【Fig. 5d】

| b | | |
|---|---|---|
| BCAM | HN1L | TSPAN13 |
| ANKHD1-EIF4EBP3 | SCN5A | CTR9 |
| RTL1 | RNASE13 | DPH3 |
| GFOD1 | SNF8 | COL13A1 |
| MMD | PALD1 | KLC2 |
| ARRB1 | RBM18 | AZI2 |
| RAVER2 | RSF1 | DGKD |
| NID1 | GKAP1 | LNPEP |
| ZNF697 | EXTL1 | ARL6IP5 |
| POMK | LOC642426 | GNPTAB |
| GPD1L | LINC00160 | SNX3 |
| SIPA1L2 | C16orf87 | SETBP1 |
| LOC100506142 | POLE4 | FERMT2 |
| PSMC6 | PIEZO1 | PDE1B |
| TMEM47 | MRPS16 | SH3PXD2B |
| ZNF717 | NUP214 | DHX29 |
| SLC44A5 | RBSN | TOM1L1 |
| LRRTM4 | FAT4 | GYG2 |
| GLTSCR1L | RHOC | MORF4L1 |
| NCKAP5 | HNRNPUL1 | FLOT2 |
| C19orf43 | AKT1 | PFKM |
| OBFC1 | CLCN6 | MMP15 |
| LOC339568 | RPS20 | ZBED6 |
| CNDP1 | APOA1BP | MECP2 |
| PABPN1 | MICAL2 | MASP1 |
| FRK | MGAT1 | SEC23A |
| ACOX2 | | |
| CDC26 | | |

【Fig. 5e】

| C | | |
|---|---|---|
| ALKBH5 | KLHL3 | SLC6A6 |
| MKRN1 | ZNF608 | PLEKHA6 |
| FHL2 | ANTXR1 | CDC37 |
| SBF2 | MPHOSPH9 | CD164 |
| FABP3 | INSIG2 | EBP |
| SNORA64 | TOMM22 | BTF3 |
| PLCXD3 | FAM210B | RPL7 |
| SEPW1 | SCAPER | UBXN2B |
| ATXN7 | IMPACT | VPS4A |
| BMP5 | VASH1 | C4A |
| EGF | KLHL31 | C4B |
| CACNA2D1 | NPPA-AS1 | HSP90B1 |
| DCAF6 | RFK | DUSP3 |
| PIEZO2 | ZNF200 | RPL3 |
| SZRD1 | ZNF467 | XRRA1 |
| SLC8A3 | PRPF31 | GTF2I |
| CACNA1I | TNNI3 | MARVELD1 |
| GXYLT1P3 | RNF150 | NPY6R |
| SDHB | PPP1R13B | ZFYVE27 |
| PCGF3 | PPP1R14C | ANKRD52 |
| SAFB | LOC101927055 | TMEM30A |
| TRIM24 | FGD5-AS1 | PRRG1 |
| PFKFB2 | FHOD3 | PKN2 |
| GUCY1A2 | LOC653513 | NEXN |
| PRICKLE1 | PSMD1 | LAMB2 |
| C6orf201 | HSPA9 | NDFIP1 |
| | SDF4 | RGS5 |

【Fig. 5f】

d MRPL19
TIMP3
SORBS2
QKI
RPL24
ANKRD1
FNDC3B
CBX6
TSC22D3
CORIN
HIPK3
RNF11
MYO18A
DES
PIK3R1
EEA1
ARG2
MYL2
EMILIN2
SIRPA
RBM24
PRKAR2B

SVEP1
SCD
STK35
SVIL
SNORD73A
BEX4
CLTB
RBM20
XPO4
LTBP1
LRRC10
TP53I11
NPPA
LANCL1
COL4A2
SOD2
SLC35F5
ARHGAP21
CMTM5
WLS
PTGFRN
TNIP1
TTN-AS1

MYL4
EPB41L3
ACTN2
FTH1
MYL9
MYOCD
ALB
IGF2BP1
ALPK3
MYH7
FYCO1
KIF13A
MYL3
SLC8A1
CTDSP2
DPYSL3
CMYA5
ALPK2
PLN
KIDINS220
TNNC1
MYL7
TNNT2
MYH6

【Fig. 6a】

**Cardiac muscle cell**

【Fig. 6b】

Ventricular muscle cell

【Fig. 7】

【Fig. 8】

Expression pattern of CD71

【Fig. 9】

CD71 - expressing cells (Day 10)

【Fig. 10】

DAPI

α-actinin

cTnT

merge

【Fig. 11】

Nodal-like cell  Atrial-like cell  Ventricular-like cell

20 mV
300 ms

Ventricular-type
(65.4%)

Atrial-type
(26.9%)

Nodal-type
(7.7%)

N=26

【Fig. 12a】

【Fig. 12b】

【Fig. 12c】

Action potential

APA: > 90 mV
APD$_{90}$: > 300 ms
Vmax: > 10 V/s

【Fig. 13】

GMP grade CMC-11 iPSC-derived CMs

~ Day 10

【Fig. 14】

【Fig. 15】

【Fig. 16】

## Ultra-low attachment plate

【Fig. 17a】

【Fig. 17b】

# Fibroblast (after thawing)

【Fig. 17c】

Cardiomyocyte (DMSO)

Live / Dead

100 μm

【Fig. 18】

【Fig. 19】

1 aggregate ≒ 41±3 cells    1 aggregate ≒ 105±5 µm

200 µm

【Fig. 20】

## Cryopreservation (6 months)    Action potential of aggregate

【Fig. 21a】

27G syringe

100 μm aggregates

【Fig. 21b】

27G syringe

Broken aggregates

200 μm aggregates

【Fig. 21c】

【Fig. 21d】

【Fig. 22a】

【Fig. 22b】

【Fig. 22c】

【Fig. 23】

【Fig. 24】

【Fig. 25】

【Fig. 26】

【Fig. 27】

【Fig. 28a】

【Fig. 28c】

Single

【Fig. 28d】

Aggregate

【Fig. 29】

【Fig. 30】

【Fig. 31】

【Fig. 32a】

【Fig. 32b】

【Fig. 32c】

【Fig. 33】

【Fig. 34】

【Fig. 35】

【Fig. 36】

Single

【Fig. 37】

Aggregate

【Fig. 38】

【Fig. 39】

【Fig. 40】

【Fig. 41】

【Fig. 42】

【Fig. 43】

【Fig. 44】

【Fig. 45】

【Fig. 46】

【Fig. 47】

## INTERNATIONAL SEARCH REPORT

| International application No. |
|---|
| **PCT/KR2020/011077** |

**A.      CLASSIFICATION OF SUBJECT MATTER**

C12N 5/077(2010.01)i; *A01N 1/02*(2006.01)i; *A61K 35/34*(2006.01)i; *A61P 9/00*(2006.01)i; C12M 3/00(2006.01)i

According to International Patent Classification (IPC) or to both national classification and IPC

**B.      FIELDS SEARCHED**

Minimum documentation searched (classification system followed by classification symbols)

C12N 5/077; A61K 35/12; A61K 35/34; C12N 5/0735; G01N 33/50; A01N 1/02; A61P 9/00; C12M 3/00

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심근세포(cardiomyocyte), 줄기세포(stem cells), CD71, 마커(marker), 심장질환 (cardiac disorder), 배양(culturing)

**C.      DOCUMENTS CONSIDERED TO BE RELEVANT**

| Category* | Citation of document, with indication, where appropriate, of the relevant passages | Relevant to claim No. |
|---|---|---|
| Y<br><br>A | KR 10-1916902 B1 (KONKUK UNIVERSITY GLOCAL INDUSTRY-ACADEMIC COLLABORATION FOUNDATION) 08 November 2018. See abstract; claims 1-3; paragraphs [0068], [0079] and [0093]; and figure 1. | 1-5,8-12<br><br>6-7,13 |
| Y | JP 2010-538665 A (CELLARTIS AB) 16 December 2010. See abstract; claims 1-9; and examples 4-6. | 1-5,8-12 |
| A | KR 10-2012-0035801 A (CHABIO&DIOSTECH CO., LTD.) 16 April 2012. See abstract; claims 1-8; paragraphs [0014]-[0022]; and examples 1-2. | 1-13 |
| A | PARK, S.-J. et al. Dual stem cell therapy synergistically improves cardiac function and vascular regeneration following myocardial infarction. NATURE COMMUNICATIONS. electronic publication 16 July 2019, vol. 10, thesis no. 3123, pp. 1-12. See entire document. | 1-13 |

☑ Further documents are listed in the continuation of Box C.     ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 December 2020** | **16 December 2020** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2020/011077** |

### C. DOCUMENTS CONSIDERED TO BE RELEVANT

| Category* | Citation of document, with indication, where appropriate, of the relevant passages | Relevant to claim No. |
| --- | --- | --- |
| A | CAI, W. et al. An Unbiased Proteomics Method to Assess the Maturation of Human Pluripotent Stem Cell-Derived Cardiomyocytes. Circulation Research. 08 November 2019, vol. 125, pp. 936-953. See entire document. | 1-13 |

Form PCT/ISA/210 (second sheet) (July 2019)

**INTERNATIONAL SEARCH REPORT**
**Information on patent family members**

International application No.

**PCT/KR2020/011077**

| Patent document cited in search report | | | Publication date (day/month/year) | Patent family member(s) | | | Publication date (day/month/year) |
|---|---|---|---|---|---|---|---|
| KR | 10-1916902 | B1 | 08 November 2018 | None | | | |
| JP | 2010-538665 | A | 16 December 2010 | AU | 2008-300897 | A1 | 26 March 2009 |
| | | | | AU | 2008-300897 | B2 | 04 December 2014 |
| | | | | CA | 2699860 | A1 | 26 March 2009 |
| | | | | CN | 101918541 | A | 15 December 2010 |
| | | | | EP | 2198012 | A1 | 23 June 2010 |
| | | | | EP | 2198012 | B1 | 31 December 2014 |
| | | | | JP | 5722628 | B2 | 27 May 2015 |
| | | | | SG | 184764 | A1 | 30 October 2012 |
| | | | | US | 2010-0278787 | A1 | 04 November 2010 |
| | | | | WO | 2009-036982 | A1 | 26 March 2009 |
| KR | 10-2012-0035801 | A | 16 April 2012 | CN | 103237887 | A | 07 August 2013 |
| | | | | US | 2013-0251691 | A1 | 26 September 2013 |
| | | | | US | 9969978 | B2 | 15 May 2018 |
| | | | | WO | 2012-047037 | A2 | 12 April 2012 |
| | | | | WO | 2012-047037 | A3 | 07 June 2012 |
| | | | | KR | 10-1364965 | B1 | 18 February 2014 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190158572

- KR 1020200091585

**Non-patent literature cited in the description**

- Search for technology for maturation induction through microenvironment control, and convergence prototype development. *Development of Advanced Medical Technology (R&D),* 23 April 2020
- Establishment of Drug Response Data for Cardiomyocytes originating from pluripotent stem cell lines of National Stem Cell Bank. *Construction of Infrastructure for National Health and Medical Research (R&D),* 16 March 2020

- Development of 3D Cardiac Microenvironment-Simulating Chip Based on Human Stem Cell-Derived Cardiomyocyte for Assessing Cardiotoxicity of Drug. *Platform Construction of New Drug Development Based on 3D Biotissue Chip (Multi-Agency)-Commercialization of 3D Biotissue Chip,* 01 April 2020